# EUROPEAN PATENT SPECIFICATION

(11) **EP 2 296 457 B1**
(45) Date of publication and mention of the grant of the patent: **19.03.2014**
(21) Application number: 09788198.1
(22) Date of filing: 16.06.2009
(51) Int. Cl.: A01G 9/00, A01H 3/00

(54) **METHOD FOR GROWING PLANTS**
PFLANZENZUCHTVERFAHREN
PROCÉDÉ DE CULTURE DE PLANTES

(30) Priority: 16.06.2008 US 139753
(43) Date of publication of application: 23.03.2011
(73) Proprietor: Holman, Edwin Henricus Antonius, 7631CN Ootmarsum (NL)
(72) Inventor: Holman, Edwin Henricus Antonius, 7631CN Ootmarsum (NL)
(74) Representative: Jansen, Cornelis Marinus
(86) International application number: PCT/NL2009/050348
(87) International publication number: WO 2010/005298

(56) References cited:
- DE-A1- 4 336 081
- US-A1- 2002 049 999
- CHEN ZHONG; GALLIE DANIEL R: "Increasing tolerance to ozone by elevating foliar ascorbic acid confers greater protection against ozone than increasing avoidance" July 2005 (2005-07), PLANT PHYSIOLOGY (ROCKVILLE), VOL. 138, NR. 3, PAGE(S) 1673-1689 , XP002547148 ISSN: 0032-0889 the whole document
- SASAKI KANAKO; SAITO TAKUYA; LAEMSAE MARI; OKSMAN-CALDENTEY KIRSI-MARJA; SUZUKI MASASHI; OHYAMA KIYOSHI; MURANAKA TOSHIYA; OHARA K: "Plants utilize isoprene emission as a thermotolerance mechanism" September 2007 (2007-09), PLANT AND CELL PHYSIOLOGY, VOL. 48, NR. 9, PAGE(S) 1254-1262 , XP002547149 ISSN: 0032-0781 the whole document
- LOIVAMAEKI MAARIA; GILMER FRANK; FISCHBACH ROBERT J; SOERGEL CHRISTOPH; BACHL ANETTE; WALTER ACHIM; SCHNITZLER JOERG-PETER: "Arabidopsis, a model to study biological functions of isoprene emission?" June 2007 (2007-06), PLANT PHYSIOLOGY (ROCKVILLE), VOL. 144, NR. 2, PAGE(S) 1066-1078 , XP002547150 ISSN: 0032-0889 the whole document
- SUGIE ATSUSHI; NAYDENOV NAYDEN; MIZUNO NOBUYUKI; NAKAMURA CHIHARU; TAKUMI SHIGEO: "Overexpression of wheat alternative oxidase gene Waox1a alters respiration capacity and response to reactive oxygen species under low temperature in transgenic Arabidopsis" October 2006 (2006-10), GENES & GENETIC SYSTEMS, VOL. 81, NR. 5, PAGE(S) 349-354 , XP002547151 ISSN: 1341-7568 the whole document
- VELIKOVA VIOLETA; PINELLI PAOLA; PASQUALINI STEFANIA; REALE LARA; FERRANTI FRANCESCO; LORETO FRANCESCO: "Isoprene decreases the concentration of nitric oxide in leaves exposed to elevated ozone" NEW PHYTOLOGIST, vol. 166, no. 2, May 2005 (2005-05), pages 419-426, XP002547152 ISSN: 0028-646X
- COJOCARIU C; HEWITT N; VICKERS C; MULLINEAUX P; VELIKOVA V: "Isoprene-induced protection in tobacco plants exposed to elevated ozone concentration" COMPARATIVE BIOCHEMISTRY AND PHYSIOLOGY PART A MOLECULAR & INTEGRATIVE PHYSIOLOGY, vol. 146, no. 4, Suppl. S, April 2007 (2007-04), pages S263-S264, XP002547153 & ANNUAL MEETING OF THE SOCIETY-FOR-EXPERIMENTAL-BIOLOGY; GLASGOW, UK; MARCH 31 -APRIL 04, 2007 ISSN: 1095-6433
- LORETO FRANCESCO; CENTRITTO MAURO; BARTA CSENGELE; CALFAPIETRA CARLO; FARES SILVANO; MONSON RUSSELL K: "The relationship between isoprene emission rate and dark respiration rate in white poplar (Populus alba L.) leaves" PLANT CELL AND ENVIRONMENT, vol. 30, no. 5, May 2007 (2007-05), pages 662-669, XP002547154 ISSN: 0140-7791
- SHARKEY THOMAS D; WIBERLEY AMY E; DONOHUE AUTUMN R: "Isoprene emission from plants: Why and how" January 2008 (2008-01), ANNALS OF BOTANY (LONDON), VOL. 101, NR. 1, PAGE(S) 5-18 , XP002547155 ISSN: 0305-7364
- ORTEGA J ET AL: "Approaches for quantifying reactive and low-volatility biogenic organic compound emissions by vegetation enclosure techniques - Part A" CHEMOSPHERE, PERGAMON PRESS, OXFORD, GB, vol. 72, no. 3, 1 June 2008 (2008-06-01), pages 343-364, XP022670678 ISSN: 0045-6535 [retrieved on 2008-02-14]
- SMEULDERS S M ET AL: "Effects of short-term ozone exposure on the carbon economy of mature and juvenile Douglas firs (Pseudotsuga menziesii (Mirb.) Franco )" NEW PHYTOLOGIST, vol. 129, no. 1, 1995, pages 45-53, XP002561126 ISSN: 0028-646X
- DATABASE WPI Week 200752 Thomson Scientific, London, GB; AN 2007-530592 XP002561127 & JP 2007 169183 A (AKUSERU KK) 5 July 2007 (2007-07-05)
- FICK JERKER ET AL: "Ozone removal in the sampling of parts per billion levels of terpenoid compounds: An evaluation of different scrubber materials" ENVIRONMENTAL SCIENCE AND TECHNOLOGY, vol. 35, no. 7, 1 April 2001 (2001-04-01), pages 1458-1462, XP002561128 ISSN: 0013-936X
- VANCANNEYT G ET AL: "CONSTRUCTION OF AN INTRON-CONTAINING MARKER GENE: SPLICING OF THE INTRON IN TRANSGENIC PLANTS AND ITS USE IN MONITORING EARLY EVENTS IN AGROBACTERIUM-MEDIATED PLANT TRANSFORMATION", MOLECULAR AND GENERAL GENETICS, SPRINGER VERLAG, BERLIN, DE, vol. 220, no. 2, 1 January 1990 (1990-01-01), pages 245-250, XP008075800, ISSN: 0026-8925, DOI: 10.1007/BF00260489

## Description

### FIELD OF THE INVENTION

The present invention is in the field of crop production. More in particular, the present invention relates to methods for growing plants and methods for increasing the average yearly crop production of a crop production area. The invention further relates to plants produced by the method of the invention exhibiting increased yield, and to the use of greenhouses comprising means for controlling the atmospheric content of a reactive oxygen species.

### BACKGROUND OF THE INVENTION

Global demand for wheat, rice, corn, and other essential grains is expected to steadily rise over the next twenty years. Apart from serving as a food source, the demands will rise as plant-based fuel and chemicals production by biological processes is growing. Meeting this demand by increasing production through increased land use is not very likely; and while better crop management may make a marginal difference, most agriculture experts agree that this anticipated deficit must be made up through increased crop yields.

Modern crop production management systems are tailored to optimize each and every parameter that influences crop yield. Management of moisture and nutrient availability and control of pests is standard in all agricultural production schemes for field crops. In greenhouse crops, availability of CO₂ and light and temperature may in addition be optimized. Hence, most crops are currently produced under apparently optimal conditions. Although some variation is perceived as "natural", realistic production efficiencies are still a fraction of theoretical maximum levels. In fact, year-average efficiency levels attained in the field for most crops are often less than half of the maximum levels observed in particular years. The reason for this difference is not properly understood. Hence, there is a need for further understanding and optimizing biological production efficiencies.

Plant biomass production is determined by the plants net carbon fixation efficiency. This efficiency is governed by the rate of carbon gain in terms of CO₂ fixation by photosynthesis and the rate of carbon loss in terms of CO₂ emission by respiration. While gross photosynthesis rises with temperature, so does respiration and whereas the photosynthesis rate tends to flatten at the optimum of the photosynthetic enzyme rubisco of about 25°C, respiration continues to rise rapidly above this temperature and roughly doubles every 10°C (Q₁₀ is usually 2). At temperatures above about 35°C (at least for C-3 species) all the sugar produced is used to support respiration. At temperatures higher than 35°C, plants respire more sugar than they can produce which leads to deterioration and ultimate death of the plant. Consequently the net photosynthesis (the production of energy compounds minus their use by respiration including photorespiration) must be considered when attempting to optimize production. As noted above, traditional methods for improving carbon balance are aimed at improving the gross photosynthesis rate. Although a relationship between respiration rate and temperature is well known, and respiration is known to vary depending on protein turnover and maintenance requirements, baseline respiration rates are generally believed to be relatively fixed.

During their life cycle, plants undergo a large number of physiological, biochemical and morphological changes that are controlled by alterations in gene expression. Yet, the morphogenetic capacity of plants is a function of both genetic and environmental parameters, of which thermal and light conditions appear to have the greatest influence. Plant ontogeny, the sum of morphogenic processes that describe the development of a plant from seed germination through to maturity, is known to influence plant production parameters, such as the degree to which plants compensate after defoliation or herbivore damage. Also, it is known that factors that affect crop growth early in ontogeny often produce modifications that extend through the season and may be manifest in altered economic yield. It is believed that this is the result of epigenetic factors, among which DNA-methylation is one of the best known. Plant ontogeny includes developmental changes in plant architecture, storage capacity, and resource allocation to different functions (e.g., storage, defence, reproduction). In the case of woody species, an increase in the plant age is associated with changes in resource allocation patterns, as the carbon/nutrient balance, storage capacity, and access to water and nutrients usually increase, while root to shoot ratio, growth rate, photosynthesis, stomatal conductance, hormone production, and metabolic activity typically decrease. In addition, morphological differences between juvenile and adult trees include variation in leaf morphology, phyllotaxy, shoot orientation, seasonal leaf retention, presence of adventitious roots, and leaf-specific mass.

It is however not known whether crop production in general is influenced by ontogenic processes, or whether factors that affect crops early in ontogeny can produce modifications that extend throughout the life of the plant.

Isoprene (2-methyl-1,3-butadiene) is emitted by a large number of plant species. Isoprene emission generally consumes a significant percentage of the carbon fixed during photosynthesis. The role of isoprene emission in plants is not fully understood. It was suggested to provide protection against heat stress. Indeed, isoprene emissions in some plants exhibit temperature response patterns that are dependent on the plant's growth temperature. Other alkenes are also found to increase thermotolerance in leaves. Isoprene is also suggested to provide a more general protection against stress conditions and in particular against (photo)-oxidative stress and it was recently shown to protect leaves against ozone. The ability of alkenes, such as isoprene, to react with singlet oxygen, ozone and OH radicals is well known, and isoprene emission has been suggested as a quencher of ozone. Thus, although the correlation between leaf temperature and isoprene emission in plants is well known, the physiological role of isoprene emission, quite costly to the plant, is still under debate.

Also vitamin C, ascorbate, is recognized as a factor that protects plants against oxidatvive stress, such as ozone stress. Further, tartaric acid and lycopene (a tetraterpene consisting of 8 isoprene units) have been proposed as a plant-derived anti-oxidants.

It is however not understood how processes for the protection against stress conditions can be made beneficiary so as to increase the yield of said plant. Hence there exists a need for processes and plants exhibiting increased yield despite the presence of yield lowering processes that are essential to plant survival.

### SUMMARY OF THE INVENTION

The present inventor has now discovered that baseline respiration rate, in particular the AOX component of the total dark respiration, is fixed early in the ontogeny of plants. In fact, the inventor has discovered that oxidative stress early in the ontogeny of plants results in plants having a high baseline respiration rate for the rest of their productive life span. Although in mature plants, the respiration rate may temporarily increase in response to altered environmental conditions, it will return to a rate the level of which is ultimately fixed at an early stage of development.

This discovery was done in Brazil, where annual sugar cane burnings result in high temporary atmospheric concentrations of ozone. It was found that plants that were planted in a period wherein ozone concentrations were lowest consistently exhibited high production, whereas plants that were planted in a period such that the early ontogenic phase coincided with these adverse conditions consistently exhibited low production rates.

Hence, based on these findings the inventor found that plants that are capable of counteracting the adverse effects of atmospheric ozone or that are prevented in any way from encountering these adverse effects during early ontogeny exhibit high production, in terms of dry matter content.

Early in ontogeny plants determine a default respiration rate based on the conditions that prevail at that time in their life. An increase in protein turnover, for instance due to increased tissue damage, will result in a higher respiration rate. It was discovered that in early ontogeny this is essentially a one-way street: the baseline respiration rate can go up, but it cannot come down. As a result, a plant that is exposed to unfavourable conditions such as high ozone concentrations that result in leaf damage and increased respiration rates early in ontogeny, will keep high baseline respiration rates essentially throughout its life, or at least for prolonged periods of time. Hence, such a plant will exhibit a low net production, as much of the assimilated carbon is respired.

The present inventor has now discovered that plants that are grown in the presence of ozone-protecting compounds, and hence that are protected from the effects of ozone (i.e oxidative stress), in particular at a certain, early, stage in their life, show an enormous increase in biomass production rates and on top of that a higher level of disease-resistance. The presence of ozone-protecting compounds can be achieved by providing specific climatological conditions in a greenhouse, or by producing these compounds in the vicinity of the plant e.g. by micro-organisms or by the plants themselves. The present inventors have discovered that levels or emission rates of ozone-protecting compounds in the vicinity of plants or in transgenic plants must be such that baseline respiration rates, in particular the AOX component thereof, are not elevated by ozone exposure. For isoprene, this is achieved when at least during early ontogeny of the plants, the isoprene emission by the plant at 30°C is at least 20 nmol·m⁻²·s⁻¹. Other suitable minimal emission rates to avoid increase in the baseline respiration rates, in particular the AOX component thereof, are for instance 5 µg/g dry weight/h, and 0.03 µg/cm²/h or 1.25 nmol·m⁻²·s⁻¹.

The mitochondrial electron transport chain involves two pathways: the cytochrome pathway and the alternative oxidase (AOX) pathway. In plants, heat stress, and as contemplated herein also drought stress, temperature stress, radiation stress, and salt stress induces the release of cytochrome c (cyt-c) from the respiratory chain, which in turn leads to elevated levels of Reactive Oxygen Species (ROS) and induction of AOX. AOX is a cyanide-resistant, hydroxamic-acid-sensitive terminal oxidase found in the inner mitochondrial membranes of plants, some fungi, and trypanosomes. The activity mediates the non-proton-translocating transfer of electrons from the UQ pool to molecular O₂ to form water. Electrons flowing through AOX bypass proton-translocating complexes III and IV of the Cyt-mediated electron transport chain, causing the oxidative potential energy to be lost as heat. The function of AOX in plants during normal vegetative growth and development remains unclear. The present inventor has now found that the minimum level of the OAX pathway as component of the total dark respiration is determined early in the life of the plant, that is: in the ontogenic phase, and that the respiration efficiency in terms of ATP generated, and therefore the yield of the plants, is less when the plants are exposed early in there life to conditions that elevate the AOX pathway. It has now also been found that this rise in the AOX pathway can be prevented intentionally, by manipulating the plant or its environment during early ontogeny. Such a manipulation is only possible on an effective level when appreciating the present invention.

In a first aspect, the present invention provides a plant as specified in the claims, preferably grown under conditions that cause periodic oxidative stress, wherein said plant or the environment of said plant was manipulated such that an increase in the rate of respiration and/or protein turnover in said plant due to said periodic oxidative stress was intentionally prevented during the early ontogeny of said plant. For instance, the plant may have been grown during its early ontogeny in the presence of a sufficient amount of ozone-protecting compound in case the periodic oxidative stress was caused by ozone. Preferably said ozone-protecting compound is produced in the vicinity of the plant, by micro-organisms or by the plant itself.

A plant of the present invention is characterized by the fact that, although it was exposed to oxidative stress-inducing conditions during its early ontogeny, exhibits an AOX as component of total dark respiration that is essentially equal to a plant of the same variety that has never been exposed to oxidative stress, and exhibits an AOX as component of total dark respiration that is significantly lower (e.g. at least 5-25% lower) than that of a plant of the same variety that was exposed to said same oxidative stress-inducing conditions during its early ontogeny but which plant or its environment were not manipulated to prevent an increase in the AOX. Moreover, the plants have increased dry matter content relative to an exposed plant which, or the environment of which, was not manipulated as indicated herein.

In another preferred embodiment of a transgenic plant of the present invention, said plant is from a species that does not naturally emit isoprene. Such a plant is herein referred to as a plant from a non-isoprene emitting plant species. Also contemplated are low-isoprene emitters, which are referred to herein as plants of which the isoprene emission rates are to be increased if an increase in the AOX during early ontogeny as a result of periodic oxidative stress is to be prevented by conversion of ozone.

In an alternatively preferred embodiment, said plant is not a transgenic Ginseng (*Panax ginseng*), Kudzu, Tobacco, Grey poplar (*Populus x canescens*), *Populus alba,* or *Arabidopsis thaliana* plant.

In a plant of the invention, said early ontogeny is the period between germination and the generative phase, thus preferably the vegetative phase, that is before the plants induce and develop flowers. This stage can be anywhere between a few days to 0.5-6 months post germination and is generally longer for trees.

In a preferred embodiment of a plant of the invention said plant exhibits a baseline respiration rate (including and preferably with reference to to the alternative oxidase pathway) as the rate of that is at most 80%, preferably at most 70, 60, 50, 40, 30, or even 20% of that of a plant which has not been subjected to ozone-protective measures as described herein, at least during early ontogeny of said plant. In a highly preferred embodiment this early ontogeny is the period 15-60 days after emergence (DAE) of the first leaf of said plant. Alternatively, in another highly preferred embodiment this early ontogeny is the period coinciding with the pre-floral stage of said plant.

In still another preferred embodiment of the plant of the invention, said plant is from a species that does not naturally produce an anti-oxidant compound mentioned above.

The plant of the invention is an adult plant, and said adult plant exhibits a first rate of respiration under ambient ozone that is essentially equal to the rate of maintenance respiration exhibited by said plant during early ontogeny, and wherein said plant upon temporary exposure to ambient plus 50 or 100 ppbv of ozone exhibits a second rate of respiration, which second rate is a significant increase relative to said first rate, and wherein following said temporary exposure said second rate of respiration returns to pre-exposure levels.

In an even more preferred embodiment of this plant said rate of maintenance respiration is the rate of respiration of a young full grown leaf of a plant measured when said plant has not been exposed to ozone, preferably said rate of respiration refers to the AOX component in de total (dark) respiration due to oxidative stress as a result of which the respiration loses efficiency in terms of generating ATP. Thus, preferably the AOX in a plant of the invention that has passed the ontogenic phase and that has been exposed to AOX-enhancing levels of ozone, is close to zero, or at least close to that of a plant never exposed to ozone or other oxidative stress. Thus, plants of the invention when placed in an oxidative-stress-free environment have the ability of having their AOX essentially return to 0% (or pre-exposure levels). Whereas plants that have not been grown by a method of the invention and that have been exposed to oxidative stress during the ontogenic phase have relatively high AOX components even when thereafter placed in an oxidative-stress-free environment.

In another aspect, the present invention provides a method for increasing the yield of a plant grown under conditions that cause periodic oxidative stress in said plant, comprising the step of growing a seedling, tissue culture or plantlet into a plant, and further comprising the step of manipulating said plant or the environment of said plant such that said periodic oxidative stress is intentionally prevented during the early ontogeny of said plant, to maintain the AOX component of the total dark respiration in said plant at levels essentially equal to that of a plant that has never been exposed to oxidative stress.

A method for increasing the yield of a plant includes reference to methods for increasing stress tolerance as indicated herein and to methods for increasing disease resistance in said plant.

In embodiments of this method, the oxidative stress is the stress in a plant that may be brought about by radiation, heat, salt, drought, SO₂, low CO₂, or reactive oxygen species, including H₂O₂, or O₃, preferably H₂O₂ or O₃, most preferably O₃ or precursors of O₃, such as NOx.

In another preferred embodiment of the method of the invention, the oxidative stress is prevented by controlling the content of a reactive oxygen species (ROS) in the growth environment. Said reactive oxygen species in the growth environment may be H₂O₂, or O₃, preferably H₂O₂ or O₃, and is most preferably O₃ in the growth atmosphere. The ROS content may be controlled in one of many ways. For instance in a closed system, it may be controlled by using ROS scrubbers. A suitable ROS scrubber is for instance an activated carbon filter. Alternatively, the ROS and particularly the O₃ content in the growth atmosphere may be controlled by exposing the plant or its growth atmosphere to isoprene. Isoprene is known to neutralize ozone. In a further preferred embodiment, the isoprene may be produced by micro-organisms (including transgenic or recombinant micro-organisms transformed with a gene encoding the enzyme isoprene synthase) in the vicinity of the plant. Alternatively, increased levels of CO₂ may be used to counteract the effects of oxidative stress during the ontogenic phase.

In yet another preferred embodiment of the method of the invention, the plant is a perennial plant and/or a crop plant, preferably a protected crop plant.

In another aspect, the present invention provides a method for increasing the average yearly crop production of a crop production area, comprising growing a plant according to a method of the invention as described above and using said plant as a crop plant in said production area.

In a preferred embodiment of this method, the increase in the average yearly crop production is brought about by an improved net carbon fixation efficiency, biomass production, dry matter content and/or pest resistance of said crop plant.

In yet another aspect, the present invention provides a plant part obtained from a plant of the present invention as described above.

In still a further aspect, the present invention provides the use of a greenhouse for growing plants to control the content of a reactive oxygen species to prevent periodic oxidative stress during early ontogeny of said plants. Preferably, said reactive oxygen species is O₃ and the greenhouse of the present invention is therefore preferably provided with 03 scrubbers optionally in combination with O₃ precursor transformers (e.g. a light source transforming NO₂ into NO + O₃).

In yet another aspect, the present invention provides a method for optimizing the production of a specific type of plant on a selected cultivated area, comprising the steps of:
a) determining for said specific type of plant the early ontogenic phase wherein the baseline respiration rate is fixed to a permanent minimum level;
b) monitoring in the air over said cultivated area the concentration of reactive oxygen species, for instance by using an atmospheric sensor capable of determining the concentration of reactive oxygen species in air; and
c) sewing a seed or planting a seedling or plantlet of said specific type of plant and allowing the seedling or plantlet to develop into a plant, wherein during said early ontogenic phase of said plant an exposure to undesirable concentrations of reactive oxygen species from the air over said cultivated area is essentially prevented by selecting the moment of sewing or planting using the data obtained in step b).

In another aspect, the present invention provides the use of an agrobiological composition comprising isoprene emitting microorganisms and an agronomically acceptable carrier, said composition optionally further comprising growth nutrients for said microorganism and substances that induce isoprene production in said microorganism, wherein said microorganism is preferably a transgenic microorganism comprising a vector for the expression of isoprene synthase, operably linked to a promoter functional in said microorganism, wherein said use is in a method for increasing the yield of a plant grown under conditions that cause periodic oxidative stress, comprising administering said composition during the early ontogeny of said plant.

In another aspect, the present invention provides the use of an agrobiological composition comprising catalase-producing microorganisms and an agronomically acceptable carrier, said composition optionally further comprising growth nutrients for said microorganism and substances that induce catalase production in said microorganism, wherein said microorganism is preferably a transgenic microorganism comprising a vector for the expression of catalase, operably linked to a promoter functional in said microorganism, and wherein said microorganism is symbiotic to said plant, wherein said use is in a method for increasing- the yield of a plant grown under conditions that cause periodic oxidative stress, comprising administering- said composition during the early ontogeny of said plant.

In another aspect, the present invention provides a method for increasing the yield of a plant grown under conditions that cause periodic oxidative stress in said plant, comprising administering during the early ontogeny of said plant the agrobiological composition of the present invention.

### SHORT DESCRIPTION OF THE DRAWINGS

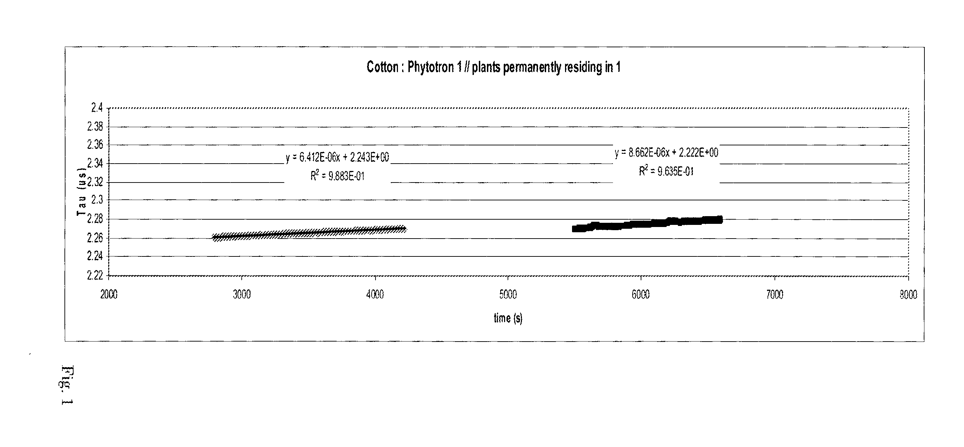

Figure 1 shows the measurement results of phase shift fluorescence measurement as described in Example 2 for cotton plants grown (and permanently residing under 0 ppbv ozone in phytotron 1 (grey bar (left): SHAM inhibited sample; black bar (right): uninhibited control).

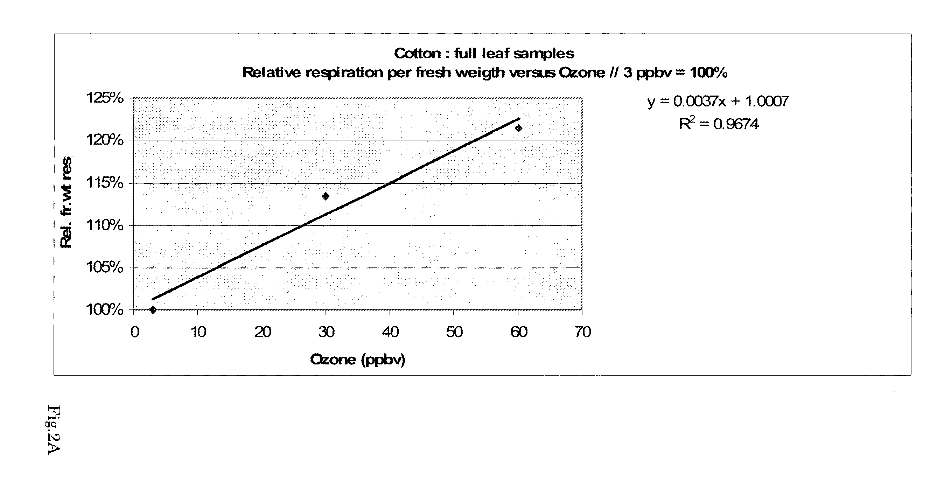

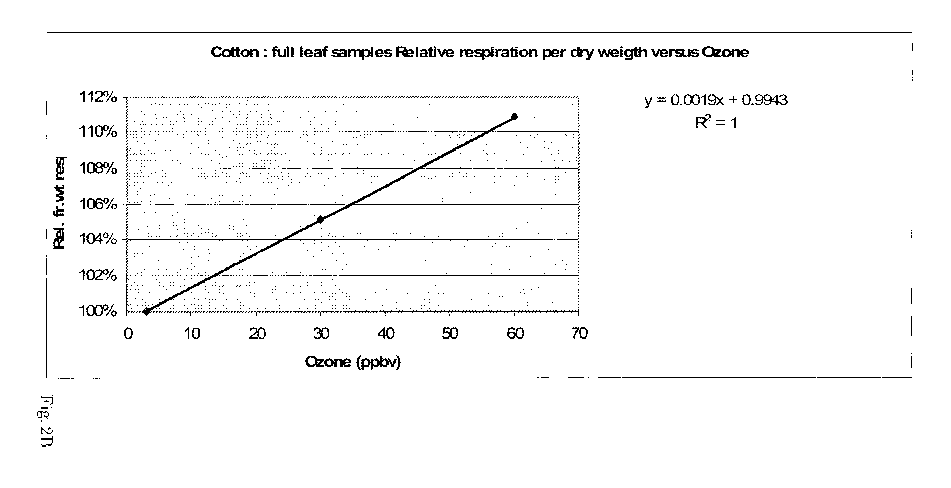

Figure 2 shows the graphic presentation of total dark respiration as measured by phase shift fluorescence as a function of ozone in Cotton full leaf samples. A: Relative respiration (TDR) per fresh weight versus Ozone // 3 ppbv = 100%. (See also Table 2); B: Relative respiration (TDR) per dry weight versus Ozone (See Table 2).

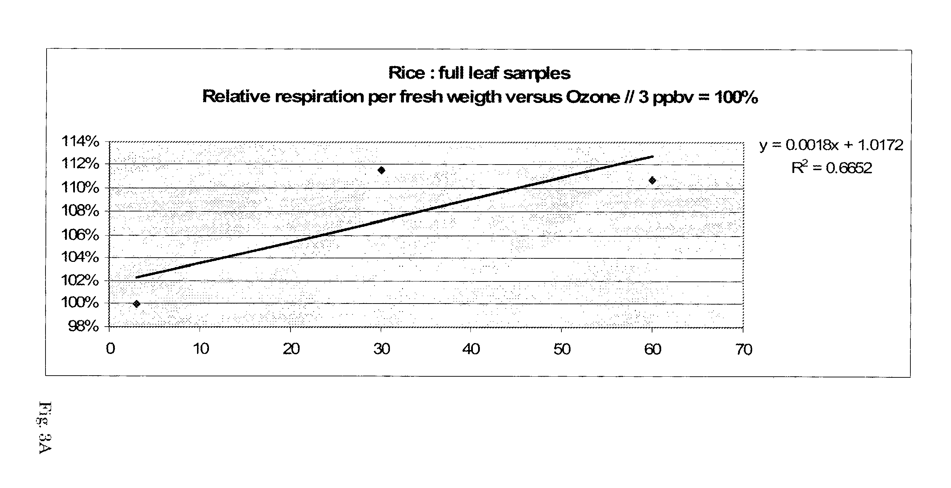

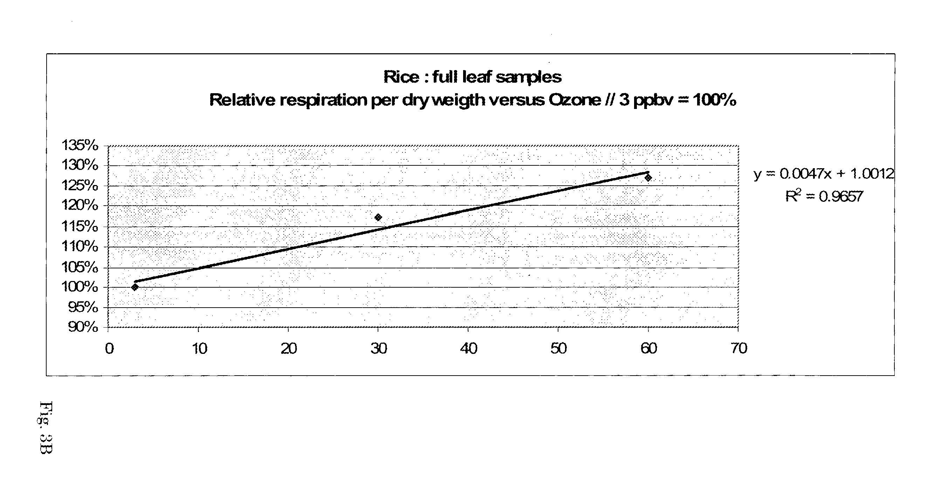

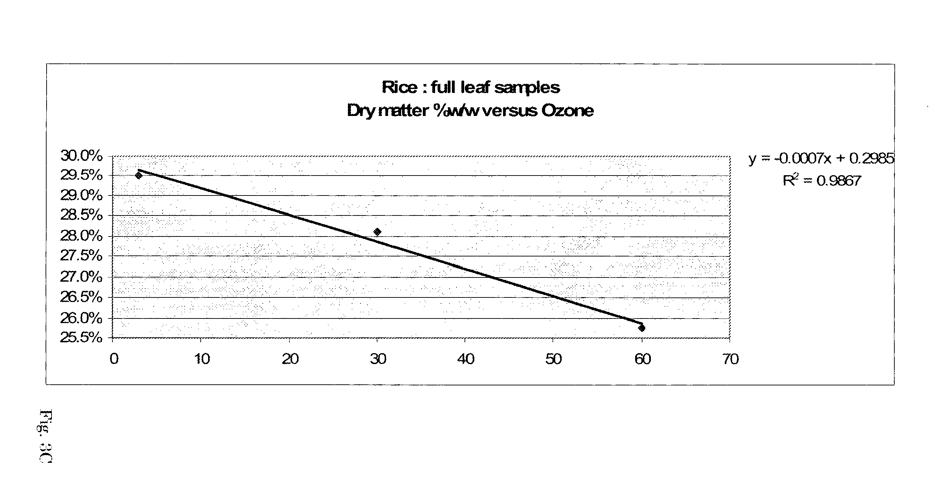

Figure 3 shows the graphic presentation of total dark respiration as measured by phase shift fluorescence as a function of ozone in Rice full leaf samples. A: Relative respiration (TDR) per fresh weight versus Ozone // 3 ppbv = 100%. (See also Table 3); B: Relative respiration (TDR) per dry weight versus Ozone (See Table 3); C: Dry matter content versus Ozone.

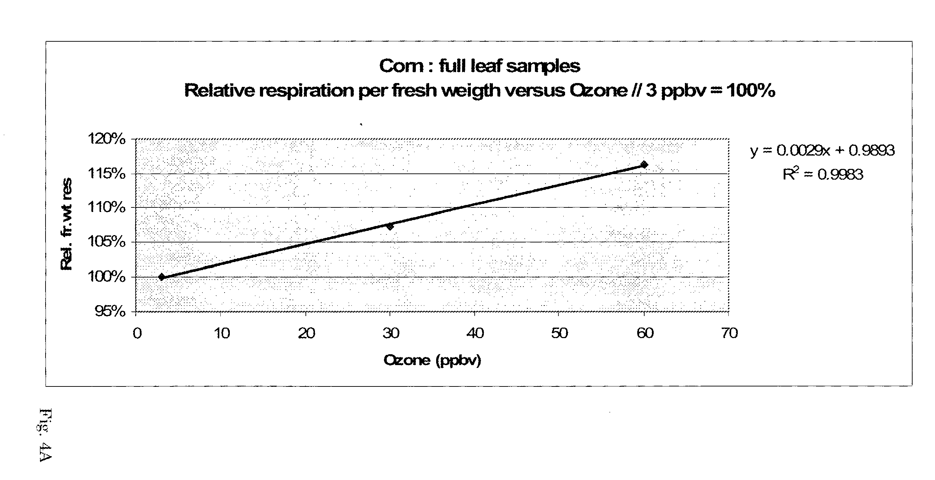

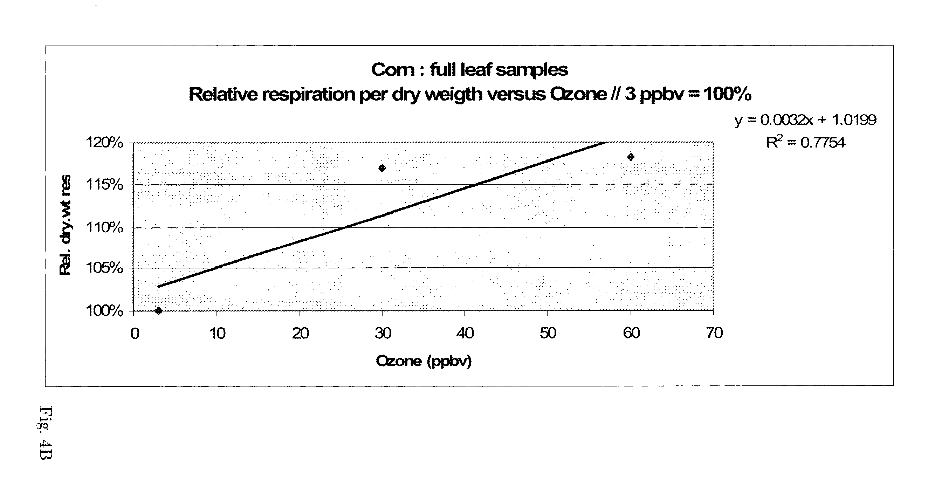

Figure 4 shows the graphic presentation of total dark respiration as measured by phase shift fluorescence as a function of ozone in Corn full leaf samples. A: Relative respiration (TDR) per fresh weight versus Ozone // 3 ppbv = 100%. (See also Table 4); B: Relative respiration (TDR) per dry weight versus Ozone (See Table 4).

### DETAILED DESCRIPTION OF THE INVENTION

### Definitions

The term "plant," as used herein, refers to any type of plant. The inventors have provided below an exemplary description of some plants that may be used with the invention. However, the list is provided for illustrative purposes only and is not limiting, as other types of plants will be known to those of skill in the art and could be used with the invention.

A common class of plants exploited in agriculture are vegetable crops, including artichokes, kohlrabi, arugula, leeks, asparagus, lettuce (e.g., head, leaf, romaine), bok choy, malanga, broccoli, melons (e.g., muskmelon, watermelon, crenshaw, honeydew, cantaloupe), brussels sprouts, cabbage, cardoni, carrots, napa, cauliflower, okra, onions, celery, parsley, chick peas, parsnips, chicory, Chinese cabbage, peppers, collards, potatoes, cucumber plants (marrows, cucumbers), pumpkins, cucurbits, radishes, dry bulb onions, rutabaga, eggplant, salsify, escarole, shallots, endive, garlic, spinach, green onions, squash, greens, beet (sugar beet and fodder beet), sweet potatoes, swiss-chard, horseradish, tomatoes, kale, turnips, and spices.

Other types of plants frequently finding commercial use include fruit and vine crops such as apples, apricots, cherries, nectarines, peaches, pears, plums, prunes, quince almonds, chestnuts, filberts, pecans, pistachios, walnuts, citrus, blueberries, boysenberries, cranberries, currants, loganberries, raspberries, strawberries, blackberries, grapes, avocados, bananas, kiwi, persimmons, pomegranate, pineapple, tropical fruits, pornes, melon, mango, papaya, and lychee.

Many of the most widely grown plants are field crop plants such as evening primrose, meadow foam, corn (field, sweet, popcorn), hops, jojoba, peanuts, rice, safflower, small grains (barley, oats, rye, wheat, etc.), sorghum, tobacco, kapok, leguminous plants (beans, lentils, peas, soybeans), oil plants (rape, mustard, poppy, olives, sunflowers, coconut, castor oil plants, cocoa beans, groundnuts), fiber plants (cotton, flax, hemp, jute), *Lauraceae* (cinnamon, camphor), or plants such as coffee, sugarcane, tea, and natural rubber plants.

Another economically important group of plants are ornamental plants. Examples of commonly grown ornamental plants include *Alsti-oemeria* (e.g., *Alstoemeria brasiliensis*), aster, azalea (e.g., *Rhododendron* sp.), begonias (e.g., *Begonia* sp.), bellflower, bouganvillea, cactus (e.g., *Cactaceae schlumbergera truncata* ), camellia, carnation (e.g., *Dianthus caryophyllus*), chrysanthemums (e.g., *Chrysanthemum* sp.), clematis (e.g., *Clematis* sp.), cockscomb, columbine, cyclamen (e.g., *Cyclamen* sp.), daffodils (e.g., *Narcissus* sp.), false cypress, freesia (e.g., *Freesia refracta*), geraniums, gerberas, gladiolus (e.g., *Gladiolus* sp.), holly, hibiscus (e.g., *Hibiscus rosasanensis*), hydrangea (e.g., *Macrophylla hydrangea*), juniper, lilies (e.g., *Lilium* sp.), magnolia, miniroses, orchids (e.g., members of the family *Orchidaceae*), petunias (e.g., *Petunia hybrida*), poinsettia (e.g., *Euphorbia pulcherima*), primroses, rhododendron, roses (e.g., *Rosa* sp.), snapdragons (e.g., *Antirrhinum* sp.), shrubs, trees such as forest (broad-leaved trees and evergreens, such as conifers) and tulips (e.g., *Tulipa* sp.).

The term "plant part", as used herein, includes reference to, but is not limited to, single cells and tissues from microspores, pollen, ovules, leaves, embryos, roots, root tips, anthers, flowers, fruits, seeds, stems, shoots, scions, rootstocks, protoplasts, calli, meristematic tissues and the like.

"Plant tissue" includes differentiated and undifferentiated tissues or plants, including but not limited to roots, stems, shoots, leaves, pollen, seeds, tumor tissue and various forms of cells and culture such as single cells, protoplast, embryos, and callus tissue. The plant tissue may be in plants or in organ, tissue or cell culture.

The term "perennial", as used herein, refers to a plant that lives for more than two years. Perennial plants can be short-lived (only a few years) or they can be long-lived, as some woody plants, such as trees.

The term "crop plant", as used herein, refers to a plant which is harvested or provides a harvestable product. Particularly preferred plants for use in aspects of the invention are protected (greenhouse) crop plants.

The terms "seedling" and "plantlet", as used herein, are interchangeable and refer to the juvenile plant grown from a sprout, embryo or a germinating seed and generally include any small plants showing well developed green cotyledons and root elongation and which are propagated prior to transplanting in the ultimate location wherein they are to mature.

The term "tissue culture", as used herein, refers to a culture of plant cells wherein the cells are propagated in a nutrient medium under controlled conditions.

"Significant increase" is an increase that is larger than the margin of error inherent in the measurement technique, preferably an increase by about 10%-50%, or even 2-fold or greater.

"Significantly less" means that the decrease is larger than the margin of error inherent in the measurement technique, preferably a decrease by about 2-fold or greater.

The term "early ontogeny", as used herein, refers to the early phase in the course of growth and development of an individual to maturity (the ontogenic phase). This phase will differ in length between plant species. For roses, this period is estimated to amount to about 6 to 8 months. In essence, the early ontogenic phase terminates when the plant characteristics in response to environmental stimuli, such as respiration rate, become established and fixed. The skilled person can determine the early ontogenic phase for any plant species by measuring the ozone-induced respiration rate in plants at periodic intervals and determining at what age of the plant the increase in respiration rate due to ozone exposure does not return to pre-exposure values, but is fixed. The ontogenic phase for many plants will be the development of the seedling trough the vegetative stage and will generally end upon entry of the generative stage (floral stage).

The term "manipulating said plant" refers to a technical intervention in said plant, *inter alia* by genetic manipulation, for instance by providing a transgenic plant as described herein, such that said plant does not increase its baseline respiration rate despite the presence of oxidative stress-inducing factors such as stress-causing ozone levels.

The term "manipulating the environment of said plant" refers to a technical intervention in the surroundings or ambient of said plant, *inter alia* by lowering the stress to said plant, for instance by providing an environment wherein the oxidative stress-inducing factors such as stress-causing ozone is reduced by technical intervention to sub-stress levels, or at least to such levels that said plant does not increase its baseline respiration rate relative to the situation without said intervention where, in the presence of oxidative stress-inducing factors such as stress-causing ozone levels, said plant would have increased its baseline respiration rate.

The term "intentionally" in the context of intentionally prevented an increase in the rate of respiration and/or protein turnover in said plant refers to the performance of an activity such as a technical intervention or a lack thereof as described above, with the purpose of maintaining the AOX component of the total dark respiration in said plant as defined herein at levels essentially equal to that of a plant that has never been exposed to oxidative stress, to thereby achieve an increase in the yield of said plant.

The term "epigenetic", as used herein, refers to the state of the DNA with respect to heritable changes in function without a change in the nucleotide sequence. Epigenetic changes can be caused by modification of the DNA, in particular chromatin remodeling caused by modifications of the histone proteins and DNA methylation. These changes affect gene transcription and ultimately affect phenotype. Epigenetic changes thus involve factors that influence behavior of a cell without directly affecting its DNA or other genetic components. The epigenetic view of differentiation is that cells undergo differentiation events that depend on correct temporal and spatial repression, derepression, or activation of genes affecting the fate of cells, tissues, organs, and ultimately, organisms. Thus epigenetic changes in an organism are normal and result in alterations in gene expression. For example, epigenetic transformation of a normal cell to a tumor cell can occur without mutation of any gene.

The term "respiration", as used herein, refers to the process by which O₂ is transported to and used by the cells and CO₂ is produced and eliminated from the cells during which process organic matter is oxidized.

The term "protein turnover", as used herein, refers to the flow of amino acids from existing protein into newly synthesized protein. Protein turnover is generally regarded as one of the most important maintenance processes in plants in terms of energy requirements. Both biosynthetic and breakdown processes affect the rate of protein turnover. Both protein synthesis and protein degradation require respiratory energy. Protein turnover has several important functions in regulating the plant's metabolism. Together with protein synthesis, degradation is essential to maintain appropriate enzyme levels and to modulate these levels based on internal and external signals. Furthermore, protein degradation is important in allowing a plant to cope with changing environmental conditions. When nutrients become limiting, the rate of protein turnover is accelerated by increasing the rate of degradation relative to synthesis, which generates a pool of free amino acids from less essential proteins that can be used to assemble more essential ones.

The term "radiation", as used herein, includes both particle radiation (ie electrons, protons), high-energy electromagnetic radiation (ie x-rays, gamma rays) and other ionizing radiation in the radiant-energy spectrum, as well as non-ionizing electromagnetic radiation, and radiation in the ultraviolet, visible light, and infra-red spectrum.

The term "periodic" in the context of "periodic stress" refers both to a discontinuous form of stress that is experienced by said plant for interrupted periods, as well as to a form of stress that is experienced by said plant for uninterrupted periods (e.g. permanently) but at varying level.

The term "oxidative stress", as used herein, refers to the state in which cells are exposed to excessive levels of molecular oxygen or reactive oxygen species (ROS) to the extent - ultimately- that damage is incurred and cellular repairs systems are mobilized. Before damage is done however, the plant cells anticipating risk to damage will upscale certain processes (e.g. by protein turnover) and/or down scales other processes (e.g. like cytoplasmic streaming) to prevent damage from occurring. This also qualifies as oxidative stress. Oxidative stress may thus be measured by increased protein turnover rates. As used herein, oxidative stress may *inter alia* be the result of drought stress, temperature stress, radiation stress, heat stress, salt stress, or reactive oxygen species. Oxidative stress as defined herein is therefore the process that increases the AOX pathway. Preventing the plant to increase its AOX pathway during the early ontogeny therefore renders such a plant less sensitive to drought stress, temperature stress, radiation stress, heat stress, salt stress. Hence methods of the present invention inherently have the result of rendering plants more resistant to other stresses than oxidative stress, including resistance to pests and disease.

The term "reactive oxygen species" (ROS), as used herein, refers to oxygen ions, free radicals, and peroxides, both inorganic and organic. They are generally very small molecules and are highly reactive due to the presence of unpaired valence shell electrons. ROSs form as a natural by-product of the normal metabolism of oxygen and have important roles in cell signalling. However, during times of environmental stress ROS levels can increase dramatically, which can result in significant damage to cell structures. This cumulates into a situation known as oxidative stress.

The term "ambient ozone" is used herein to indicate an ozone level of about 10 to 15 ppbv to about 35 ppbv. The level may vary between night and day and may be about 0 ppbv at night and about 45 to 120 ppbv during the day.

The term "growth environment", as used herein, refers to the soil, substrate, solution or air in which the plant is growing.

The term "scrubber", as used herein with reference to reactive oxygen species or O₃ (ozone) scrubber, refers to a system, device, or chemical capable of binding or chemically converting reactive oxygen species, such as O₃ to the effect that said reactive oxygen species is eliminated from the environment which is placed in contact with the scrubber.

The term "isoprene", as used herein, refers to the chemical compound 2-methylbuta-1,3-diene.

The term "lycopene", as used herein, refers to the chemical compound (6E, 8*E*,10*E*,12*E*,14*E*,16*E*,18*E*,20*E*,22*E*,24*E*,26*E*)-2,6,10,14,19,23,27,31-octamethyldotriaconta-2,6,8,10,12,14,16,18,20,22,24,26,30-tridecaene, and is a tetraterpenoid compound, assembled from 8 isoprene units.

The term "endogenous" as in "endogenously produced" refers to produced within the plant (cell).

The term "isoprene synthase", as used herein, refers to the enzyme with registry number EC 2.5.1.-, that catalyses the elimination of pyrophosphate from dimethylallyl diphosphate to form isoprene. The amino acid sequence of the enzyme in *Populus alba* is provided in SEQ ID NO:1. The term is considered to cover homologues having >80%, preferably >90% amino acid identity with SEQ ID NO:1, and in particular refers to homologues that produce isoprene from dimethylallyl diphosphate.

The term "prefloral stage", as used herein, refers to the ontogenic stage preceding the emergence of reproductive structures in a plant.

The term "post-germination", as used herein, refers to the period in the development of the plant following the emergence of the radicle from the seed.

The term "average yearly crop production", as used herein, refers to the amount of crop produced per annum or season (average seasonal crop production) in the form of weights or number of plants or plant parts harvested or weight gain in crop biomass (fresh and or dry weight) are expressed per unit of production area, and wherein the individual amounts per annum for multiple years are summated and divided by the number of years.

The term "production area", as used herein, refers to a location where plants are grown and where products in the form of plants or plant parts are produced for harvest. The size of the production area is generally expressed in square meters or acres of land. A production area can be an open field or a greenhouse.

The term "net carbon fixation efficiency", as used herein, is used interchangeable with the term "net photosynthetic efficiency" and refers to the net efficiency with which carbon dioxide is converted into organic compounds, taking into account the losses due to respiration.

The term "biomass production", as used herein, refers to the production of plant derived organic material.

The term "dry matter content", as used herein, refers to the mass fraction (%) that remains after the water fraction (%) has been removed by drying.

The term "pest resistance", as used herein, refers to resistance against viral, bacterial, fungal, and insect pests, as well as pests by molluscs and nematodes.

The term "greenhouse", as used herein, refers to any structure comprising walls, a roof, and a floor designed and used principally for growing plants in a controlled and protected environment. The walls and roof are usually constructed of transparent or translucent material to allow passage of sunlight for plant growth.

### Methods of Growing Plants

The methods of growing plants according to the present invention are based on three essential realizations:
i) respiration rates reached in a plant during early ontogeny culminate in the development of a baseline respiration rate, which is the minimum rate at which said plant will respire when mature;
ii) this baseline respiration rate is attained by one or more step-up increments and is ultimately fixed at baseline level by epigenetic changes;
iii) the step-up increments in the respiration rate are the plant's response to increased protein turnover rates which in turn are the result of oxidative stress experienced by said plant, and
iv) the step-up increments in the AOX component of the total dark respiration are the result of oxidative stress experienced by said plant.

Therefore, when attempting to keep the respiration rates in mature plants as low as possible, it is important to minimize the oxidative stress to said plant during the phase wherein the baseline respiration rate is fixed by epigenetic factors, that is, early in ontogeny.

Hence, a method of growing plants according to the present invention comprises the important step of allowing a seedling, tissue culture or plantlet to develop into a plant and to pass through its early ontogenic phase wherein epigenetic factors fix the baseline respiration rate to a permanent minimum level. The early ontogenic phase wherein epigenetic factors fix the baseline respiration rate to a permanent level can differ for different plants, and can therefore not be defined for each and every plant species in advance. Also, the phase at which epigenetic factors determine the permanent minimum rate of respiration may vary between plant varieties within a species. This phase may however be experimentally determined for each and every plant species or plant variety as follows:

Seedlings, tissue cultures or plantlets are allowed to develop into mature plants. The total development time is recorded and divided into a large but practical number of regular intervals (between 2 and for instance 10, 20, 50, 100, or 1000 intervals). During each interval, the respiration rate of the developing plant is measured using standard techniques. After each measurement, the plant is temporarily subjected to oxidative stress, for instance by exposing it to ozone. The oxidative stress can optionally be applied at incrementally increasing levels during the different intervals. The early ontogenic phase wherein epigenetic factors fix the baseline respiration rate to a permanent level is now determined from the data obtained as:
a) the total number of intervals preceding the interval in which the respiration rate returns to a baseline level after said oxidative stress period, or
b) the total number of intervals during which the respiration rate shows a step-wise increase relative to the preceding interval and no decrease in the subsequent or following interval.

Roughly, in a method of the present invention the early ontogenic phase wherein epigenetic factors fix the baseline respiration rate to a permanent level is the prefloral stage, preferably a period from 0.1-6 months, preferably 0.5-6 months following the germination.

A method of growing plants according to the present invention further comprises the important step of preventing during early ontogeny of said plant the occurrence of an undesirable increase in the rate of respiration and/or protein turnover due to oxidative stress. It is to be understood that in accordance with the above-described model for epigenetic fixation of baseline respiration rates, an undesirable increase in the rate of respiration is equivalent to an undesirable increase in the rate of protein turnover.

Oxidative stress is often, but not exclusively, the result of damage to the plant brought about by radiation or reactive oxygen species. Hence, the prevention of an undesirable increase in the rate of respiration and/or protein turnover due to oxidative stress can be attained by preventing exposure to radiation or reactive oxygen species that result in such an increase. Most notably, intracellular H₂O₂ levels and atmospheric O₃ concentrations are maintained at sub-stress levels. Such levels may differ between plant species and between plant varieties, but can be easily determined experimentally. For instance, a plant can be exposed to a certain test level of radiation or reactive oxygen species for a predetermined period of time (i.e. varying between several minutes to several weeks) and the rate of respiration and/or protein turnover is determine before and after said exposure. A sub-stress level of radiation or reactive oxygen species is the level at which the exposure does not result in an increase of the post-exposure rate relative to the rate before.

Thus, in preferred embodiments of methods of the present invention, the oxidative stress is prevented by controlling the content of a reactive oxygen species in the growth environment such that they remain at sub-stress levels.

The growth environment may be the soil, substrate, medium or atmosphere in which the plant is grown. Preferably the content of the reactive oxygen species, most preferably O₃, is controlled in the growth atmosphere.

In order to prevent oxidative stress, the O₃-content of the growth atmosphere of the plant during the early ontogenic phase is preferably kept below 100 ppbv, most preferably below 75 ppbv, even more preferably below 60 ppbv, still more preferably below 50 ppbv, even more preferably below 40 ppbv, yet even more preferably below 35, 30, 25, 20, 15, 10, 5 or 1 ppbv, wherein ppbv refers to parts per billion by volume. Sporadic increases are likely not to have a major effect on the plants, yet values above 100 ppbv should essentially be avoided at all times during the early ontogenic phase.

Essentially, this may be attained in different ways. For instance, the content in the total airspace (atmosphere) in which the plant is grown may be controlled. Control in the total airspace will generally involve the use of closed cultivation systems, such as greenhouses. Effective control of the level of reactive oxygen species can be obtained by the use of scrubbers or air filtering systems. Such filtering systems are well known in the field of conditioning of air. Suitable scrubbers or filter materials include those materials that absorb or adsorb reactive oxygen species. An example of a suitable material is activated carbon, but other materials capable of filtering reactive oxygen species from air that is passed through the filter of over the filter material are also suitable. It is preferred that an air circulation system is used wherein the air is moved over or through the scrubber. Also suitable is the use of a material that reacts with the reactive oxygen species in the atmosphere, thereby rendering the reactive oxygen species unreactive. A suitable example of such a material is isoprene. Hence, a greenhouse may periodically be loaded with isoprene gas in order to remove reactive oxygen species from the air. Generally, an amount of isoprene equivalent to 1-1000, more preferably 50-150 parts per billion by volume (ppbv) of air is sufficient.

Alternatively, only the content in the air that is in direct contact with the plant surface may be controlled. This is most effectively attained by providing the plant with an exogenous source of isoprene. To this end, isoprene may be provided in the form of a vapour in the growth atmosphere of the plant, or at least at the leaves of a plant. Alternatively, isoprene may be produced by epiphytes such as microorganisms including bacteria, fungi and yeasts living in symbioses with the plant at its surface (leaf, stem or root).

Alternatively, only the content in the air in the endogenous free space (that also difuses out into to the atmosphere) may be controlled, e.g. by endophytes such as microorganisms including bacteria, fungi and yeasts living in symbioses within the plant.

Alternatively, only the ROS content, specifically H₂O₂, in the endogenous liquid free layer may be controlled, e.g. by catalase-positive endophytes, such as microorganisms including bacteria, fungi and yeasts living in symbioses within the plant and being capable of liberating the H₂O₂ converting enzyme catalase.

Methods of growing plants according to the present invention have as an advantage that the net biosynthetic efficiency of said plant is increased during its entire lifetime. The invention is therefore especially important for plants that have a life cycle of multiple years, because it is in these plants that a high baseline respiration rate attained during early ontogeny has the most impact on the total production of the plant over its entire lifetime. Hence a plant in aspects of the present invention is preferably a perennial plant.

It will also be appreciated that the methods of the present invention are particularly suitable for plants in which production parameters are very important, such as for instance in crop plants and plants projected to help counteracting atmospheric CO₂ accumulation for climate stabilizing purposes (carbon credits).

A method of growing a plant according to the present invention is preferably practiced on a large number of plants simultaneously as a part of crop production. Hence, in another aspect, the present invention provides a method for increasing the average yearly crop production of a crop production area, comprising growing a plant of the invention or according to a method of the invention as described above and using said plant as a crop plant in said production area.

It is an advantage of the plants and methods of the present invention that the average yearly crop production is no longer adversely affected by seasonal fluctuations in the atmospheric load of reactive oxygen species.

A crop production area can be a small field of a few square metres, but most preferably is a large production area covering several acres or even a large number of square miles. The advantages of the present invention are best appreciated when a large number of plants are involved. The methods of the present invention can be performed on large cultivated areas, and can for instance comprise the monitoring of environmental factors that influence oxidative stress, and selecting the planting or sewing season such that in its early ontogenic phase as defined herein the plant is exposed to a minimum of oxidative stress. Thus, the method of the invention can be made part of a crop cultivation system comprising the monitoring of for instance atmospheric reactive oxygen species, and using this information to select the optimal planting or sewing season for the crop such that the crops are not exposed to undesirable or stress-level concentrations of atmospheric reactive oxygen species such as O₃.

Based on the teachings of the present invention, the skilled person will understand that certain planting seasons in Brazil and other parts of the world that suffer from high ozone levels during certain parts of the year, are best avoided due to excessive atmospheric concentrations of reactive oxygen species, such as during sugar cane burning. It is an aspect of the present invention that such avoidance can be managed effectively by monitoring the concentration of reactive oxygen species in the air over a cultivated area using atmospheric pollution sensors or "sniffers" for determining the concentration of reactive oxygen species in air.

Therefore in another aspect, the present invention provides a method for optimizing the production of a specific type of plant on a selected cultivated area, comprising the steps of:
a) determining for said specific type of plant the early ontogenic phase wherein epigenetic factors fix the baseline respiration rate to a permanent level, for instance by using methods as described above;
b) monitoring for said selected cultivated area the concentration of reactive oxygen species in the air over said cultivated area, for instance by using an atmospheric sensor capable of determining the concentration of reactive oxygen species in air, or sampling air and determining the concentration in said sample; and
c) sewing a seed or planting a seedling or plantlet of said specific type of plant and allowing the seedling or plantlet to develop into a plant, wherein during early ontogeny of said plant an increase in the rate of respiration and/or protein turnover due to oxidative stress as a result of reactive oxygen species in the growth atmosphere is essentially prevented, by selecting the moment of sewing or planting using the data obtained in step b).

It should be understood that the monitoring in step b) need not be constant but must be frequent enough to allow the determination of the optimal moment for sewing or planting. The optimal moment for sewing or planting is the moment that assures that the subsequent early ontogenic phase wherein the baseline respiration rate is fixed to its minimum level coincides with the lowest oxidative stress induction, such induction being the result of the minimum sum of all oxidative stress inducing factors present including the concentrations of reactive oxygen species in the growth atmosphere.

When reference is made herein to terms such as optimizing the production or increase in the average yearly crop production, it is meant that such attributes include or are brought about by an improved net carbon fixation efficiency, biomass production, dry matter content and/or pest resistance of said crop plant.

Another aspect of the present invention is the use of a greenhouse for growing plants, in particular by methods of the present invention. The greenhouse comprises means for controlling the content of a reactive oxygen species in the greenhouse atmosphere. Preferably, said greenhouse is equipped with a scrubber for removing reactive oxygen species from the growth environment. In addition, the greenhouse may be equipped with systems or devices for monitoring the concentration of reactive oxygen species in the greenhouse atmosphere (i.a. the air inside the greenhouse), for instance in the form atmospheric sensors capable of determining the concentration of reactive oxygen species in air.

### EXAMPLES

### Example 1

As one example, the present invention may be performed as follows. In a greenhouse roses are grown under normal conditions of temperature, water, nutrients and light. In addition, the greenhouse atmosphere is controlled for the amount of ozone, for instance by using an ozone scrubber in order to reduce the amount of ozone in the greenhouse atmosphere. Furthermore, in order to avoid entry of ozone from the outside atmosphere, the greenhouse is well closed.

At least for the duration of the early ontogenic period as defined herein above, the ozone levels in the greenhouse are maintained at the lowest possible levels, preferably below 20-30 ppbv. Thereafter, that is after the early ontogenic phase, the plants may be grown under less stringent conditions or the plants may be brought outside the greenhouse for further growth.

The exposure to the beneficial growth environment will result in plants having a lower respiration rate when mature - compared to plants being exposed to high ozone environments, and such plants will exhibit higher production output as described herein above.

### Example 2

The Examples as described below demonstrate that the ontogenic phase of plants as described herein provides for a memory effect with respect to respiration via the AOX pathway. In particular it will be shown that the ontogenic phase is a learning period during which oxidative stress, such as provoked by ozone, irreversibly upregulates the non ATP rendering Alternative Oxidative Pathway or AOX component within the total dark respiration in ambients with higher ozone levels. Respiration via this AOX pathway is the "alternative oxidative pathway respiration", which is also referred to as "alternative path respiration" (APR), "cyanide insensitive respiration" or "SHAM sensitive respiration".

The tests, which were carried out in 3 ambients with different ozone levels, show that cotton plants at the end of their vegetative phase and upon entering the generative phase (i.e. in the ontogenic phase) have a defined and fixed maximum respiration efficiency expressed as [TDR-AOX]/[TDR], wherein TDR is the total dark respiration, and wherein AOX is the alternative oxidative pathway (AOX) respiration.

This respiration efficiency is set by the amount or level of ozone to which the plant is exposed in its ontogenic phase, herein referred to as the ontogenic phase level of ozone (OPLO). After the ontogenic phase, exposure of the plants to ozone levels higher than OPLO increases the AOX (AOX follows and upregulates). When the ozone level is subsequently returned to OPLO, the AOX also returns to the initial ontogenic level. Exposure of the plants to ozone levels lower than OPLO does not result in a lowering of the AOX (i.e. the AOX doesn't follow but maintains it's original level established during the ontogenic phase).

It was found that plants with higher respiration efficiency also have higher leaf weight (both when expressed as fresh and as dry weight) per surface area.

### Materials and Methods

### Plant material

The following plants were used in the experiments: cotton (*Gossypium* spp.; dicotyledonous), bean (*Phaseolus vulgaris* L.; dicotyledonous), corn (*Zea mays* L.; monocotyledonous), sugarcane (*Saccharum* spp.; monocotyledonous), rice (*Oryza sativa* L.; monocotyledonous), soy (*Glycine max* L.; dicotyledonous) and wheat *Triticum aestivum* L; monocotyledonous). Cotton, bean, corn, rice, soy and wheat were provided in the form of seeds. Cane was provided in the form of plantlets derived from tissue culture clones grown in agar.

### Planting

The seeds were planted in standard PE plastic flower vases no.15 (height 15 cm, upper internal diameter of 15.5 cm, bottom internal diameter of 12 cm, with eight 10mm drainage holes in the bottom). At the bottom of each vase a round piece of aquarium filter cloth of 12 cm diameter was placed in order to prevent gravel stones to wash out of the pot. 480 ml of washed stone gravel has been placed on top of the filter cloth. A second piece of filter cloth was placed on top of the gravel in order to separate it from the next layer above, and prevent the mixing of the two layers. The second layer consisted of 1.30 litres of a mixture of perlite and vermiculite (50/50 % v/v), pre-saturated with 570 ml of nutrient solution (see below).

For all crops except sugar cane 8 selected seeds were planted per pot at the following depths: Cotton: 2 cm; beans: 2 cm; corn: 4 cm; rice: 1.5 cm; soy: 3 cm depth; wheat 2.5 cm. All pots were covered with a dome consisting of a transparent (PP) 1 litre vase turned upside down. The rim of this cover vase was neatly in contact with the inner wall of the planting pot. The seeds were allowed to germinate by placing the pots in the dark at 30 °C. Cotton, beans and corn germinated first and were placed inside a phytotron three days after planting of the seeds and domes were removed the next day. Rice, soy and wheat germinated later and were placed inside a phytotron 5 days after planting with removal of the domes the next day.

For sugarcane 4 plantlets per pot were provided and these plantlets were transplanted from the agar following washing of their root system with nutrient solution (see below) in order to remove residual agar. Every sugar cane pot was provided with a dome consisting of a 5 litre water flask (PP) of which the bottom was removed creating a 30 cm high dome. Crêpe-tape was used to provide an air-tight connection between the bottom rim of the flask and the planting pot. The 40 mm screw cap on top functioned as a ventilation valve, initially maintaining near 100% RV to prevent the plantlets from drying out. Directly after transplanting, the sugar cane plantlets were placed in a phytotron (4 pots per phytotron) more in particular in acrylic cabinets created therein (see below). The sugar cane domes were removed 10 days after transplanting.

### Nutrient solution.

A single formulated nutrient solution was given to all crops during the full testing period containing 15 meq/l cations and 15 meq/l anions giving it an EC of 1.7 mS/cm. pH = 5.8. Macro nutrients: K = 4.50 mmol/l, Ca = 3.75 mmol/l, Mg = 1.50 mmol/l, N-NO3 = 9.75 mmol/l, N-NH4 = 0 mmol/1, SO4 = 1.95 mmol/l, PO4 = 1.35 mmol/l. Micro nutrients: B = 28 µmol/l, Zn-EDTA = 13 µmol/l, Cu-EDTA = 1.6 µmol/l, Mo = 0.1 µmol/l, Fe-EDDHMA = 52 µmol/l, Mn-EDTA = 12 µmol/l. On all instances where water was required only distilled water was used. The nutrient solution was the only water source for the plants.

### Growth conditions

**Phytotron.** In order to create and maintain the desired climate in an ambient that physically supports the plants to be exposed and tested, three identical phytotrons were constructed. Each phytotron was designed in order to internally control: light level, air temperature, air humidity, air CO₂ level, air O₃ level and air circulation. Steel plated, electrostatically painted white modules with 50mm and 100mm thick isopore insulation designed for cold store construction were used to create three identical and hermetically sealed rooms of 4.0 m by 3.0 m by 2.5 m (l×w×h) = 30 m³. Each room was equipped with a single sealed door for access.

**Acryl Growing Cabinets.** Inside each phytotron two fully transparent growing cabinets (90 cm (deep) x 120 cm (wide) x 140 cm (heigh)) were provided (5-6 mm transparent acrylic plates). Each growing cabinet was further divided into four plant-compartments with acrylic plates, each plant compartment housing four pots with test plants. Each plant-compartment was vertically separated from a neighbouring compartment by a lamp-compartment housing 4 tube lights (Philips TLD 30W/75, 2000 lumen & 25 µmol/s) vertically positioned one above the other. The outside of the outermost plant-compartments was also fitted with a lamp-compartment for complete illumination of the compartment. A total of 40 Philips TLD 30W/75 lamps were installed in each phytotron. Each plant-compartment was further fitted with four port holes (22 x 45 cm) that could be closed. The cabinets were positioned on a metal base giving them a ground clearance of 60cm. The top of each cabinet was provided with a mirror facing inward to reflect upgoing light.

**Light Control:** A Programmable Lighting Control (PLC) (model Logo 12/24 RC from Siemens) was used to control the photoperiod and lamp sequence. The acrylic cabinets were equipped with four levels of light counting 10 lamps each (40 lamps in total), numbered 1-4 from top to bottom. Level 1 was activated at 5:00 a.m. In sequence every next level came in one hour later providing full light at 8:00 a.m. At 8:00 p.m. all levels were deactivated at once providing full darkness.

**Anti-chamber.** A sealed central corridor gave access to the phytotrons and had the function of anti-chamber. The central corridor was kept at negative pressure in order to minimize air mixing between the phytotrons in the event of opening their doors. A blower fan at the end of the corridor was used to further minimize ozone translocation from high ozone to low ozone regime climates by moving the air from the lower ozone chambers to the higher ozone levels (e.g. from phytotron 1 (set point = 0 ppbv O₃) towards phytotron 2 (set point = 30 ppbv O₃) towards phytotron 3 (set point = 60 ppbv O₃)).

**CO₂ Control**: One central CO₂ interface & control system for CO₂ control was used (C-Control II, Conrad Electronic GmbH, Germany).

At 10 minutes interval the CO₂ concentration in each of the three phytotrons as well as in the outside ambient air was measured using a CO₂ analyzer (Priva 250E, (NDIR) gas analyzer, Priva, Holland).

Set point for CO₂ in each phytotron was set at 420 ppmvat 940 mBar and 26 °C during the photoperiod and 450 ppmvat 940 mBar and 26 °C for the dark period.

Before being interpreted, CO₂ measurements were pressure- and temperature-compensated by the central CO₂ interface & control system, corrected for atmospheric pressure variations, temperature variations in and outside the phytotrons and friction losses in the guiding tubes, electric selenoids and drying filters interconnecting the phytotrons with the central CO₂ interface & control system. The system allows for a 20 ppmvCO₂ hysteresis. In order to justify air renovation in case of excess internal CO₂ concentrations, principally during the dark period, outside air was also being evaluated and was taken to be at least 20 ppmvbelow the internal excess concentration for the system to activate the air exhaust system. In case of sub set point CO₂ concentrations, principally due to photosynthesis activity during the photoperiod, pure CO₂ was injected. The amount of CO₂ required was calculated and its addition was controlled by the central CO₂ interface & control system. Every phytotron was internally equipped with a security solid state CO₂ monitor (CDM4161A module, Figaro, Japan), which triggered an alarm at CO₂ levels over 1000 ppmv.

**Internal air circulation.** Two internal circulation ventilators (model Master Fan Top, from Treviso Brazil) were used to ensure circulation of 15.5 m3 of air per minute in the growing cabinets.

**Air Exhaust System.** An air exhaust system was used to lower the CO₂ concentration in phytotron by partially substituting the air in the phytotron with outside air. An axial blower fan (model Taurus-H from Ventisilva Brazil) was used to drive the exchange whereby an external particles pre filter (model SA-3085 from Inpeca Brazil) and six activated carbon gas-mask chemical end filters (model RC203 from Carbografite Brazil) were used in combination to guarantee the incoming air quality.

**Temperature control.** York, high wall split cooling system, model YJDA-12FS-ADA with 3.52 kW cooling capacity was used. Temperature was set to average 26 °C (+/- 1.5 °C) after air-passage through the growing cabinets.

**Humidity control.** Inside each phytotron, a ventilated psychrometer ((Engineering dep. Crops Advance Brazil) was used to monitor its wet and dry bulb temperature in a micro-fan boosted forced air stream. At a temperature difference equal to or more than 3.0 °C, the psychrometer activated two piezo-element water nebulizers (model Polaris Ion 35W from Mallory of which the internal high tension "ion" generators have been removed) until the temperature difference restores to 2.0 °C or less. This translated to vapor pressure deficit values between 4 to 8 mBar and relative humidity values between 75% to 85% within a temperature interval of 22 °C being close to the evaporater unit outlet, to 27 °C at the outlet of the acryl growing cabinets. Condensation water draining from the evaporator unit inside each phytotron was captured for recirculation. It was passed through a UV-C sterilization unit (Atman UV-5W equipped with a Philips TUV PL-S 5W UV-C lamp) before being collected in a 40 litres collection reservoir. The collection reservoir was equipped with a submerged pump (Atman HF-750 18W) to supply the interconnected water reservoirs of the nebulizers with water in order to guarantee minimum water levels. At minimum level, an electronic level control system activates the pump. At full capacity, the collection reservoir drains into an external jerry-can.

**Ozone control.** Ozone generators (model OP-35-3P, Grupo Interozone, Brazil) were coupled to ozone monitors (model 202, 2B Technologies, Inc. USA; dynamic range of 1.5 ppbv to 100 ppmv of ozone; precision 1.5 ppbv or 2% of the reading) using a ozone monitor - generator interface (Engineering dep. Crops Advance Brazil). At full light level (all 40 lamps activated) and no reactive surfaces other than the internal physical phytotron structure present to interfere, the background ozone level inside the phytotrons did not exceed 3 ppbv ozone. This was used as the upper limit for phytotron number one. Phytotron number two was set at 30 ppbv average ozone. Phytotron number three was set at 60 ppbv average ozone. Ozone levels of phytotron number two and three were permanently measured at 10 second intervals and averaged per minute. Each ozone monitor fed averaged values over an analog output to an ozone monitor - ozone generator interface. Hysteresis was set at +/- 5 ppbv. Ozone levels were monitored close to the inlet of the two internal circulation ventilators. The ozone generators were adapted to accept an external digital signal from the interface.

**Closed circuit video camera system.** Every phytotron was equipped with a video camera (Panasonic SDR-S7) sending its images over a TecVoz TEC30/04LIG2 digital video record system to a server CPU, recording all images. Over the test period the cameras have been manually put into position to record the plants in test.

### Respiration // Gas exchange measurements

Respiration of plant material was tested as follows: Partial oxygen pressure drops due to oxygen consumption as result of dark respiration were measured using phase shift fluorescence measurement. For this, a NeoFox™ Phase Measurement System was used (Fluorometer Bench version using a QBIF600-VIS-NIR laboratory-grade bifurcated optical fiber with FOXY-R-8cm overcoated fluorescence oxygen needle sensor, Ocean Optics Inc. USA).

Fluorescence phase shift was expressed as Tau in microseconds (µs) and has an inverse and linear relation with the partial oxygen pressure. The sensor had an atmospheric volume fraction gain of O₂ per -1µs Tau = 23.79% at 26.0 °C and 936 mBar. An exemplary graph of cotton is presented in Figure 1

Salicylhydroxamic acid (SHAM) (99%, Sigma-Aldrich Brasil Ltda. São Paulo, Brazil) was used as an AOX inhibitor. The soluble sodium salicylhydroxamate salt was prepared by titrating SHAM with 0.1 M sodium hydroxide until neural pH. A stock solution (0.136 M) was prepared containing 300 ml/L ethanol from initial dissolving SHAM (same abbreviation is used for the salicylhydroxamate salt). From the stock solution a 20 mmol/L salicylhydroxamate final test solution was prepared by taking 12.3 ml stock solution and completing till 100 ml with a phosphate buffer solution (pH 7). The final SHAM test solution thus contained 3.7% residual ethanol. For proper control, the comparative test solution was also provided with 3.7 ml of ethanol per 100 ml of phosphate buffer solution (pH 7). To both solutions two drops of wetting agent (Silwet L-77 AG, GE Silicones Inc. USA) were added per 25 ml of test solution.

Plant material collected was always the first fully grown leaf counting from the apical point downwards or outwards (e.g. for monocotyls). For cotton this resulted in the third leaf under the apical point. Per test, four leafs from four different plants were sampled. Cotton leafs were cut in half along the mid nerve. Using a clipper, 16 leaf sections of equal surface area of 400.9 mm2 each were cut, one from each base half and one from each top half part giving 4 sections per leaf and 16 sections per test. The leaf sections were distributed equally over two sub samples, one with and one without salicylhydroxamate. Using a chirurgical blade, all sections were given 7 parallel cuts (through and through) at approximately 2.5 mm apart over almost the full length of the section to guarantee lateral infiltration of the test solutions and gas exchange.

Within 10 minutes of plant sampling the sections were cut, initial subsample weight was registered using a high precision laboratory scale and the subsamples were placed in their solutions with and without salicylhydroxamate for 60 minutes in the dark, to guarantee dark adaptation.

Each sample contained 4 leaves, 4 sections per leaf providing 16 sections, 8 per subsample. In 1 subsample of 8 sections AOX was inhibited, the other subsample served as control. Thereafter each subsample material was tissue dried and stacked using filter paper sections cut with the same clipper to separate individual leaf sections. Each subsample consisted of 9 filter sections and 8 leaf sections. Each stacked subsample was placed inside a sterile 50 ml normal tip syringe (Embramac, Campinas Brazil) and the internal volume was set at 10 ml bruto.

### Respiration // Gas exchange measurements

The drop in partial oxygen vapour pressure due to oxygen consumption as result of dark respiration was measured using phase shift fluorescence measurement.

Both syringes (SHAM and control) were placed in a 16 litres temperature-stabilized insulated water bath with forced water circulation equipped with a temperature control unit (C-Control II, from Conrad Electronic GmbH, Germany) using a high precision temperature sensor interface set at 26.00 °C and maintaining the water temperature within +/- 0.03 °C by calculating and applying the necessary heat injection through a 25W glass coated resistor element (Engineering dep. Crops Advance Brazil).

The oxygen needle probe from the NeoFox™ Phase Measurement System was inserted into the tip of the 50 mL syringe, sealed and the complete setup was submerged in the water bath. The syringes were given 60 minutes of temperature stabilization before valid readings were taken. The SHAM sample was read first and readings were taken every second. The readings in general tended to linearly stabilize within 15 to 20 minutes. Up to 20 minutes of stable trajectory was registered before switching the syringes and the stabilizing and reading processes were repeated for the non-SHAM subsample.

Between measurements of the SHAM and non-SHAM subsample, the probe was allowed to stabilize. The readings were collected into computer memory. From this, the Total Dark Respiration (TDR) including the AOX (non-SHAM) and Total Dark Respiration (TDR) excluding the AOX (SHAM) were determined. The results for cotton are provided in Table 1.

**Table 1. Total dark respiration and salicyl hydroxamate (SHAM)-sensitive AOX as measured by Phase shift fluorescence measurement - Cotton**

| | TDR excl AOX | TDR incl AOX | AOX component | AOX/TDR | AOX/TDR |
|---|---|---|---|---|---|
| Situation | dTau/t (µs/s)⁸ without SHAM | dTau/t (µs/s)⁸ with SHAM | (arb units) | | relative to initial equilibrium |
| 0 pphv ozone¹ | 6.41E-06 | 8.66E-06 | 2.25 | 26.0% | 1.00.0% |
| 0 →30 ppbv ozone² | 7.51E-06 | 1.08E-05 | 3.26 | 30.3% | 116.5% |
| 0 →60 ppbv ozone³ | 5.75E-06 | 9.52E-06 | 3.77 | 39.6% | 152.5% |
| 0 →60 →0 ppbv ozone⁴ | 8.87E-06 | 1.18E-05 | 2.95 | 25.0% | 96.2% |
| 30 ppbv ozone⁵ | 6.96E-06 | 9.93E-06 | 2.97 | 29.9% | |
| 60 ppbv ozone⁶ | 6.03E-06 | 9.36E-06 | 3.33 | 35.6% | 100.0% |
| 60 →0 ppbv ozone⁷ | 7.13E-06 | 1.11E-05 | 3.97 | 35.8% | 100.5% |

| | | | | | |
|---|---|---|---|---|---|
| ¹plants grown in 0 ppvb O₃; ²planets grown in 0 ppvb O₃ and transferred to 30 ppvb O₃; ³plants grown in 0 ppvb O₃ and transferred to 60 ppvb O₃; ⁴plants grown in 0 ppvb O₃, transferred to 60 ppvb O₃ and transferred back to 0 ppvb O₃; ⁵plants grown in 30 ppvb O₃; ⁶plants grown in 60 ppvb O₃; plants grown in 60 ppvb O₃ and transferred to 0 ppvb O₃. ⁸ Values are the slope of the measurement curve as indicated in Fig. 1 | | | | | |

In addition, full leaf Total Dark Respiration (TDR) measurements without the use of any pre-treatment solution (no SHAM and pretreatment control) were executed for cotton, corn and rice. Data (tables 2, 3 and 4) show clear positive correlation between a) total respiration per unit of fresh weight and ozone level, and b) total respiration per unit of dry weight and ozone level.

**Table 2. Full leaf Cotton Total Dark Respiration (TDR) measurements**

| | | | |
|---|---|---|---|
| Ozone concentration during growth (ppbv.) | 0 | 30 | 60 |
| Normalized dark respiration activity (fwt)¹ | 82.4 | 72.6 | 67.9 |
| Normalized clark respiration activity (dwt)² | 14.1 | 13.4 | 12.7 |

| | | | |
|---|---|---|---|
| ¹ As h/Tau/g fresh weigth/40ml. ² As h/Tau/g dry weigth/40ml | | | |

**Table 3. Full leaf Rice Total Dark Respiration (TDR) measurements**

| | | | |
|---|---|---|---|
| Ozone concentration during growth (ppbv) | 0 | 30 | 60 |
| Normalized dark respiration activity (fwt)¹ | 32.6 | 29.2 | 29.4 |
| Normalized dark respiration activity(dwt)² | 9.6 | 8.2 | 7.6 |

| | | | |
|---|---|---|---|
| ¹ As h/Tau/g fresh weigth/40ml. ² As h/Tau/g dry weigth/40ml | | | |

**Table 4. Full leaf Corn Total Dark Respiration (TDR) measurements**

| | | | |
|---|---|---|---|
| Ozone concentration during growth (ppbv) | 0 | 30 | 60 |
| Normalized dark respiration activity (fwt)¹ | 83.9 | 78.3 | 72.1 |
| Normalized dark respiration activity (dwt)² | 17.2 | 14.7 | 14.5 |

| | | | |
|---|---|---|---|
| ¹As h/Tau/g fresh weigth/40ml. ² As h/Tau/g dry weigth/40ml | | | |

### Conclusions

The plants that passed their ontogenic phase in the 0 ppbv ozone ambient have an AOX respiration rate of 26.0% of the total 100% Total Dark Respiration rate. When placed in the 30 ppbv ozone ambient, the AOX respiration increased to 30.3%, +16.5% compared to the initial AOX respiration rate and those placed in the 60 ppbv ozone ambient increased their AOX respiration to 39.6%, +52.5% compared to the initial AOX respiration rate.

When returned to the 0 ppbv ozone ambient in which the plants lived their ontogenic phase, the AOX respiration of the plants which had passed time in the 60 ppbv ozone ambient returned fully to its initial level.

Plants grown up in the 60 ppbv ozone level ambients did not lower their AOX respiration during their stay in the zero ozone level ambient and maintained their high AOX respiration rate.

The AOX respiration rate expressed as percentage of the total dark respiration is defined as the base level during the ontogenic phase.

Plants with AOX levels defined during the ontogenic phase increase their AOX respiration when exposed to oxidative stress induced by higher ozone levels, but when the oxidative stress is reduced, cotton showed its AOX respiration rate to fully return to base level within 3 days. When exposed to ozone levels lower than the base level of the plants, the AOX respiration does not go below the base level defined during the ontogenic phase.

These findings have great implications for methods for preventing inefficient respiration in plants, and hence for increasing plant yield.

## Claims

1. A method for increasing the yield of a plant grown under conditions that cause periodic oxidative stress in said plant, comprising the step of growing a seedling, tissue culture or plantlet into a plant, and further comprising the step of manipulating said plant or the environment of said plant such that said periodic oxidative stress is intentionally prevented during the early ontogeny of said plant to maintain the AOX component of the total dark respiration in said plant at levels essentially equal to that of a plant that has never been exposed to oxidative stress.

2. Method according to claim 1, wherein said conditions that cause periodic oxidative stress are selected from periodic drought stress, periodic temperature stress, periodic radiation stress, periodic salt stress and periodic O₃ exposure.

3. Method according to claim 1 or 2, wherein said AOX component of the total dark respiration in said plant is close to zero.

4. Method according to any one of claims 1-3, wherein said oxidative stress is intentionally prevented by controlling the content of a reactive oxygen species in the growth environment.

5. Method according to any one of claims 1-4, wherein the periodic oxidative stress during the early ontogeny of said plant is prevented by exposing the plants to a level of CO₂ that reduces the rate of respiration.

6. Method according to claim 4 or 5, wherein said reactive oxygen species in the growth environment is O₃ in the growth atmosphere.

7. Method according to any one of claims 3-6, wherein said O₃ exposure is controlled by using O₃ scrubbers and/or NOx scrubbers.

8. Method according to any one of claims 3-6, wherein said O₃ exposure is controlled by exposing said plant or its growth atmosphere to exogenously or endogenously produced isoprene, preferably wherein said isoprene is exogenously produced by optionally transgenic or recombinant microorganisms capable of emitting isoprene.

9. Method according to any one of claims 1-8, wherein the early ontogeny of said plant is the prefloral stage or the vegetative stage, preferably a period from 1-6 months post-germination.

10. Method according to any one of claims 1-9, wherein said plant is a perennial plant or a crop plant, most preferably wheat, corn, soy, potato, rice, sugarcane, sugarbeet, evening primrose, meadow foam, hops, jojoba, peanuts, safflower, barley, oats, rye, wheat, sorghum, tobacco, kapok, beans, lentils; peas, soybeans, rape, mustard, poppy, olives, sunflowers, coconut, castor oil plants, cocoa beans, groundnuts, cotton, flax, hemp, jute, cinnamon, camphor, coffee, sugarcane, tea, and a natural rubber plant.

11. Method according to any one of claims 1-10, wherein said method further comprises the step of discontinuing the manipulation of said plant or the environment of said plant when the ontogenic phase of said plant has ended.

12. Method according to any one of claims 1-11, wherein said method further comprises determining prior to or simultaneously to growing said seedling, tissue culture or plantlet into a plant:
- the total dark respiration and/or respiration via the alternative oxidase (AOX) pathway in a plant of the same variety;
- the length of the ontogenic phase in a plant of the same variety; and/or
- the length and interval of the periodic conditions that cause oxidative stress; and using said information in order to intentionally prevent exposure of said plant to said periodic oxidative stress during the early ontogeny of said plant, but not during the post-ontogenic phase, preferably wherein said information is used to select the sowing or (trans)planting date for said plant in order to essentially prevent exposure of said plant to said periodic oxidative stress during the early ontogeny of said plant.

13. Method according to any one of claims 1-12, wherein said the early ontogeny of said plant is the vegetative or prefloral phase, and wherein the post-ontogenic phase is the generative phase.

14. Method for increasing the average yearly crop production of a crop production area, comprising growing a plant according to any one of claims 1-13 and using said plant as a crop plant in said production area, preferably wherein said increase in the average yearly crop production is brought about by an improved net carbon fixation efficiency, biomass production, dry matter content and/or pest resistance of said crop plant.

15. A plant obtainable by the method according to any one of claims 1-13, said plant exhibiting a baseline respiration or a respiration via the alternative oxidase (AOX pathway that is significantly less than a plant of the same variety grown under the same periodic conditions that cause oxidative stress but not grown by the method of any one of claims 1-13, wherein said plant is an adult plant, and wherein said plant exhibits a first rate of respiration under ambient ozone that is essentially equal to the rate of dark respiration exhibited by said plant during early ontogeny, and wherein said plant upon temporary exposure to ambient plus 100 ppbv of ozone exhibits a second rate of respiration, which second rate is a significant increase relative to said first rate, and wherein following said temporary exposure said second rate of respiration returns to pre-exposure levels, preferably wherein said early ontogeny is the period between germination and 0.5-6 months post germination or wherein said early leaf ontogeny is the period between emergence and 15-60 days after emergence of the first leaves.

16. Plant according to claim 15, wherein the plant is a non-isoprene emitter or a low-isoprene emitter.

17. Plant according to any one of claims 15-16, wherein said plant is a perennial plant or a crop plant, most preferably wheat, corn, soy, potato, rice, sugarcane, sugarbeet, evening primrose, meadow foam, hops, jojoba, peanuts, safflower, barley, oats, rye, wheat, sorghum, tobacco, kapok, beans, lentils, peas, soybeans, rape, mustard, poppy, olives, sunflowers, coconut, castor oil plants, cocoa beans, groundnuts, cotton, flax, hemp, jute, cinnamon, camphor, coffee, sugarcane, tea, and a natural rubber plant

18. Plant according to any one of claims 15-17, wherein said plant exhibits a rate of respiration via the alternative oxidase (AOX) pathway that is below 30, preferably below 28, 27, 26 or 25% of the total dark respiration.

19. A plant part obtained from a plant according to any one of claims 15-18, said plant part exhibiting a baseline respiration or a respiration via the alternative oxidase (AOX) pathway that is significantly less than a plant part of a plant of the same variety grown under the same periodic conditions that cause oxidative stress but not grown by the method of any one of claims 1-13, wherein said plant part exhibits a first rate of respiration under ambient ozone that is essentially equal to the rate of dark respiration exhibited by the plant from which the part is obtained during early ontogeny, and wherein said plant part upon temporary exposure to ambient plus 100 ppbv of ozone exhibits a second rate of respiration, which second rate is a significant increase relative to said first rate, and wherein following said temporary exposure said second rate of respiration returns to pre-exposure levels, preferably wherein said early ontogeny is the period between germination and 0.5-6 months post germination or wherein said early leaf ontogeny is the period between emergence and 15-60 days after emergence of the first leaves.

20. Use of a greenhouse for growing plants to control the content of a reactive oxygen species to prevent periodic oxidative stress during early ontogeny of said plants, preferably wherein said reactive oxygen species is O₃.

21. Use according to claim 20, wherein said greenhouse is provided with O₃ scrubbers to maintain the AOX component of the total dark respiration in said plant at levels essentially equal to that of a plant that has never been exposed to oxidative stress.

22. A method for optimizing the production of a specific type of plant on a selected cultivated area, comprising the steps of:
a) determining for said specific type of plant the early ontogenic phase wherein the baseline respiration rate or the rate of respiration via the alternative oxidase (AOX pathway is fixed to a permanent minimum level;
b) monitoring in the air over said cultivated area the concentration of reactive oxygen species; and
c) sewing a seed or planting a seedling or plantlet of said specific type of plant and allowing the seedling or plantlet to develop into a plant, wherein during said early ontogenic phase of said plant an exposure to undesirable concentrations of reactive oxygen species from the air over said cultivated area is essentially prevented by selecting the moment of sewing or planting using the data obtained in step b).

23. Use of an agrobiological composition in a method for increasing the yield of a plant grown under conditions that cause periodic oxidative stress in said plant, comprising administering said agrobiological composition during the early ontogeny of said plant, wherein said agrobiological composition comprises isoprene emitting microorganisms, said composition further comprising growth nutrients for said microorganism and substances that induce isoprene production in said microorganism, wherein said microorganism is preferably a transgenic microorganism comprising a vector for the expression of isoprene synthase, operably linked to a promoter functional in said microorganism.

24. Use of an agrobiological composition in a method for increasing the yield of a plant grown under conditions that cause periodic oxidative stress in said plant, comprising administering said agrobiological composition during the early ontogeny of said plant, wherein said agrobiological composition comprises catalase-producing microorganisms, said composition further comprising growth nutrients for said microorganism and substances that induce catalase production in said microorganism, wherein said microorganism is preferably a transgenic microorganism comprising a vector for the expression of catalase, operably linked to a promoter functional in said microorganism, and wherein said microorganism is symbiotic to said plant.

25. Method for increasing the yield of a plant grown under conditions that cause periodic oxidative stress in said plant, comprising administering during the early ontogeny of said plant an agrobiological composition comprising isoprene emitting microorganisms, said composition optionally further comprising growth nutrients for said microorganism and substances that induce isoprene production in said microorganism, wherein said microorganism is preferably a transgenic microorganism comprising a vector for the expression of isoprene synthase, operably linked to a promoter functional in said microorganism.

26. Method for increasing the yield of a plant grown under conditions that cause periodic oxidative stress in said plant, comprising administering during the early ontogeny of said plant an agrobiological composition comprising catalase-producing microorganisms, said composition optionally further comprising growth nutrients for said microorganism and substances that induce catalase production in said microorganism, wherein said microorganism is preferably a transgenic microorganism comprising a vector for the expression of catalase, operably linked to a promoter functional in said microorganism, and wherein said microorganism is symbiotic to said plant.

## Patentansprüche

1. Verfahren zum Erhöhen des Ertrags einer Pflanze, angebaut unter Bedingungen, die bei der Pflanze periodischen oxidativen Stress verursachen, umfassend den Schritt, dass ein Setzling, eine Gewebekultur oder ein Pflänzchen zu einer Pflanze gezüchtet wird, und ferner umfassend den Schritt, dass die Pflanze oder die Umgebung der Pflanze so manipuliert wird, dass der periodische oxidative Stress absichtlich während der frühen Ontogenese der Pflanze verhindert wird, um die AOX-Komponente der Atmung in absoluter Dunkelheit der Pflanze auf Stufen zu halten, die im Wesentlichen denen einer Pflanze entsprechen, die nie oxidativem Stress ausgesetzt war.

2. Verfahren nach Anspruch 1, wobei die Bedingungen, die periodischen oxidativen Stress verursachen, ausgewählt sind aus periodischem Dürrestress, periodischem Temperaturstress, periodischem Strahlungsstress, periodischem Salzstress und periodischer O₃-Exposition.

3. Verfahren nach Anspruch 1 oder 2, wobei die AOX-Komponente der Atmung bei völliger Dunkelheit in der Pflanze nahe null ist.

4. Verfahren nach einem der Ansprüche 1-3, wobei der oxidative Stress durch Kontrolle des Inhalts einer reaktiven Sauerstoffspezies in der Wachstumsumgebung absichtlich verhindert wird.

5. Verfahren nach einem der Ansprüche 1-4, wobei der periodische oxidative Stress während der frühen Ontogenese der Pflanze durch Exposition der Pflanzen an eine CO₂-Stufe, welche die Atmungsrate vermindert, verhindert wird.

6. Verfahren nach Anspruch 4 oder 5, wobei die reaktive Sauerstoffspezies in der Wachstumsumgebung das O₃ in der Wachstumsatmosphäre ist.

7. Verfahren nach einem der Ansprüche 3-6, wobei die O₃-Exposition durch Verwendung von O₃-Abscheidern und/oder NOx-Abscheidern kontrolliert wird.

8. Verfahren nach einem der Ansprüche 3-6, wobei die O₃-Exposition durch Exponieren der Pflanze oder deren Wachstumsatmosphäre gegenüber exogen oder endogen produziertem Isopren kontrolliert wird, wobei das Isopren vorzugsweise exogen durch optional transgene oder rekombinante Mikroorganismen, die in der Lage sind, Isopren zu emittieren, produziert wird.

9. Verfahren nach einem der Ansprüche 1-8, wobei die frühe Ontogenese der Pflanze das präflorale Stadium oder das vegetative Stadium ist, vorzugsweise ein Zeitraum von 1-6 Monaten nach der Keimung.

10. Verfahren nach einem der Ansprüche 1-9, wobei die Pflanze eine mehrjährige Pflanze oder eine Erntepflanze ist, am meisten bevorzugt Weizen, Mais, Soja, Kartoffel, Reis, Zuckerrohr, Zuckerrübe, Nachtkerze, Sumpfblume, Hopfen, Jojoba, Erdnuss, Florasafran, Gerste, Hafer, Roggen, Weizen, Hirse, Tabak, Kapok, Bohnen, Linsen, Erbsen, Sojabohnen, Raps, Senf, Mohn, Oliven, Sonnenblumen, Kokosnuss, Kastorölpflanzen, Kakaobohnen, Erdnüsse, Baumwolle, Flachs, Hanf, Jute, Zimt, Kampfer, Kaffee, Zuckerrohr, Tee und eine Naturkautschukpflanze.

11. Verfahren nach einem der Ansprüche 1-10, wobei das Verfahren ferner den Schritt umfasst, bei dem die Manipulation der Pflanze oder der Umgebung der Pflanze eingestellt wird, wenn die ontogene Phase der Pflanze beendet ist.

12. Verfahren nach einem der Ansprüche 1-11, wobei das Verfahren ferner das Bestimmen folgender Aspekte vor oder gleichzeitig mit dem Wachstum des Setzlings, der Gewebekultur oder des Pflänzchens zu einer Pflanze umfasst:
- die Atmung in völliger Dunkelheit und/oder die Atmung über den Weg der alternativen Oxidase (AOX) in einer Pflanze derselben Sorte;
- die Dauer der ontogenen Phase bei einer Pflanze derselben Sorte; und/oder
- die Dauer und das Intervall der periodischen Bedingungen, die oxidativen Stress verursachen;
und die Verwendung der Information, um absichtlich die Exposition der Pflanze an den periodischen oxidativen Stress während der frühen Ontogenese der Pflanze zu verhindern, aber nicht während der post-ontogenen Phase, wobei die Information bevorzugt verwendet wird, um das Datum für das Säen- oder (Um)pflanzen der Pflanze zu wählen, damit im Wesentlichen die Exposition der Pflanze an den periodischen oxidativen Stress während der frühen Ontogenese der Pflanze verhindert wird.

13. Verfahren nach einem der Ansprüche 1-12, wobei die frühe Ontogenese der Pflanze die vegetative oder präflorale Phase ist und wobei die post-ontogene Phase die generative Phase ist.

14. Verfahren zur Erhöhung der durchschnittlichen jährlichen Ernteproduktion eines Ernteproduktionsgebiets, umfassend den Anbau einer Pflanze nach einem der Ansprüche 1-13 und die Verwendung der Pflanze als eine Erntepflanze in dem Produktionsgebiet, vorzugsweise wobei die Erhöhung der durchschnittlichen jährlichen Ernteproduktion durch eine Verbesserung von Netto-Kohlenstofffixierungseffizienz, Biomasseproduktion, Trockenmassegehalt und/oder Schädlingsresistenz der Erntepflanze bewerkstelligt wird.

15. Pflanze, erhältlich durch das Verfahren nach einem der Ansprüche 1-13, wobei die Pflanze eine Basislinien-Atmung oder eine Atmung über den Weg der alternativen Oxidase (AOX) aufweist, die signifikant weniger ist als bei einer Pflanze derselben Sorte, angebaut unter denselben periodischen Bedingungen, die oxidativen Stress verursachen, aber nicht angebaut durch das Verfahren nach einem der Ansprüche 1-13, wobei die Pflanze eine erwachsene Pflanze ist und wobei die Pflanze eine erste Atmungsrate unter Umgebungsozon aufweist, die im Wesentlichen gleich der Rate der Dunkelatmung ist, welche die Pflanze während der frühen Ontogenese aufweist, und wobei die Pflanze bei temporärer Exposition an Umgebungsozon plus 100 ppbv eine zweite Atmungsrate aufweist, die eine signifikante Erhöhung in Bezug auf die erste Rate ist und wobei im Anschluss an die temporäre Exposition die zweite Atmungsrate auf die Stufen vor der Exposition zurückkehrt, vorzugsweise wobei die frühe Ontogenese die Periode zwischen Keimung und 0,5-6 Monaten nach der Keimung ist oder wobei die frühe Blatt-Ontogenese die Periode zwischen dem Erscheinen und 15-60 Tage nach dem Erscheinen der ersten Blätter ist.

16. Pflanze nach Anspruch 15, wobei die Pflanze ein Nicht-Isopren-Emitter oder ein Niedrig-Isopren-Emitter ist.

17. Pflanze nach einem der Ansprüche 15-16, wobei die Pflanze eine mehrjährige Pflanze oder eine Erntepflanze ist, besonders bevorzugt Weizen, Mais, Soja, Kartoffel, Reis, Zuckerrohr, Zuckerrübe, Nachtkerze, Sumpfblume, Hopfen, Jojoba, Erdnuss, Florasafran, Gerste, Hafer, Roggen, Weizen, Hirse, Tabak, Kapok, Bohnen, Linsen, Erbsen, Sojabohnen, Raps, Senf, Mohn, Oliven, Sonnenblumen, Kokosnuss, Kastorölpflanzen, Kakaobohnen, Erdnüsse, Baumwolle, Flachs, Hanf, Jute, Zimt, Kampfer, Kaffee, Zuckerrohr, Tee und eine Naturkautschukpflanze.

18. Pflanze nach einem der Ansprüche 15-17, wobei die Pflanze eine Atmungsrate über den Weg der alternativen Oxidase (AOX) aufweist, die unter 30, bevorzugt unter 28, 27, 26 oder 25 % der Atmung bei völliger Dunkelheit ist.

19. Pflanzenteil, erhalten von einer Pflanze nach einem der Ansprüche 15-18, wobei der Pflanzenteil eine Basislinien-Atmung oder eine Atmung über den Weg der alternativen Oxidase (AOX) aufweist, die signifikant weniger ist als bei einem Pflanzenteil einer Pflanze derselben Sorte, angebaut unter denselben periodischen Bedingungen, die oxidativen Stress verursachen, aber nicht angebaut durch das Verfahren nach einem der Ansprüche 1-13, wobei der Pflanzenteil eine erste Atmungsrate unter Umgebungsozon aufweist, die im Wesentlichen gleich der Rate der Dunkelatmung ist, welche die Pflanze, von welcher der Pflanzenteil erhalten wurde, während der frühen Ontogenese aufweist, und wobei der Pflanzenteil bei temporärer Exposition an Umgebungsozon plus 100 ppbv eine zweite Atmungsrate aufweist, die eine signifikante Erhöhung in Bezug auf die erste Rate ist und wobei im Anschluss an die temporäre Exposition die zweite Atmungsrate auf die Stufen vor der Exposition zurückkehrt, vorzugsweise wobei die frühe Ontogenese die Periode zwischen Keimung und 0,5-6 Monaten nach der Keimung ist oder wobei die frühe Blatt-Ontogenese die Periode zwischen dem Erscheinen und 15-60 Tage nach dem Erscheinen der ersten Blätter ist.

20. Verwendung eines Gewächshauses zum Anbauen von Pflanzen, um den Anteil einer reaktiven Sauerstoffspezies zu kontrollieren, um periodischen oxidativen Stress während der frühen Ontogenese der Pflanzen zu verhindern, vorzugsweise wobei die reaktive Sauerstoffspezies O₃ ist.

21. Verwendung nach Anspruch 20, wobei das Gewächshaus mit O₃-Abscheidern versehen ist, um die AOX-Komponente der Atmung bei völliger Dunkelheit der Pflanze auf Stufen zu halten, die im Wesentlichen gleich denen einer Pflanze sind, die nie oxidativem Stress ausgesetzt war.

22. Verfahren zum Optimieren der Produktion eines spezifischen Pflanzentyps auf einem ausgewählten Anbaugebiet, umfassend folgende Schritte:
a) Bestimmen für den spezifischen Pflanzentyp der frühen ontogenen Phase, wobei die Basislinien-Atmungsrate oder die Atmungsrate über den Weg der alternativen Oxidase (AOX) auf eine permanente Mindeststufe festgelegt ist;
b) Überwachen in der Luft über dem Anbaugebiet der Konzentration reaktiver Sauerstoffspezies; und
c) Säen eines Samens oder Pflanzen eines Setzlings oder Pflänzchens des spezifischen Pflanzentyps und Ermöglichen des Heranwachsens des Setzlings oder Pflänzchens zu einer Pflanze, wobei während der frühen ontogenen Phase der Pflanze eine Exposition an unerwünschte Konzentrationen reaktiver Sauerstoffspezies aus der Luft über dem Anbaugebiet im Wesentlichen verhindert wird, indem der Zeitpunkt des Säens oder Pflanzens unter Verwendung der in Schritt b) erhaltenen Daten gewählt wird.

23. Verwendung einer agrobiologischen Zusammensetzung in einem Verfahren zur Erhöhung des Ertrags einer Pflanze, angebaut unter Bedingungen, die periodischen oxidativen Stress bei der Pflanze verursachen, umfassend das Verabreichen der agrobiologischen Zusammensetzung während der frühen Ontogenese der Pflanze, wobei die agrobiologische Zusammensetzung Isopren-emittierende Mikroorganismen umfasst, die Zusammensetzung ferner Wachstumsnährstoffe für die Mikroorganismen und Substanzen umfasst, die die Isopren-Produktion in den Mikroorganismen herbeiführt, wobei der Mikroorganismus bevorzugt ein transgener Mikroorganismus ist, umfassend einen Vektor zur Expression der Isopren-Synthase, funktionsfähig gebunden an einen funktionellen Promoter in dem Mikroorganismus.

24. Verwendung einer agrobiologischen Zusammensetzung in einem Verfahren zur Erhöhung des Ertrags einer Pflanze, angebaut unter Bedingungen, die periodischen oxidativen Stress bei der Pflanze verursachen, umfassend die Verabreichung der agrobiologischen Zusammensetzung während der frühen Ontogenese der Pflanze, wobei die agrobiologische Zusammensetzung Katalase-produzierende Mikroorganismen umfasst, die Zusammensetzung ferner Wachstumsnährstoffe für die Mikroorganismen und Substanzen umfasst, die die Katalase-Produktion bei dem Mikroorganismus herbeiführen, wobei der Mikroorganismus bevorzugt ein transgener Mikroorganismus ist, umfassend einen Vektor für die Expression von Katalase, funktionsfähig gebunden einen funktionellen Promoter in dem Mikroorganismus, und wobei der Mikroorganismus symbiotisch zu der Pflanze ist.

25. Verfahren zur Erhöhung des Ertrags einer Pflanze, angebaut unter Bedingungen, die periodischen oxidativen Stress bei der Pflanze verursachen, umfassend das Verabreichen während der frühen Ontogenese der Pflanze einer agrobiologischen Zusammensetzung, umfassend Isopren-emittierende Mikroorganismen, wobei die Zusammensetzung ferner Wachstumsnährstoffe für die Mikroorganismen und Substanzen umfasst, die die Isopren-Produktion in den Mikroorganismen herbeiführen, wobei der Mikroorganismus bevorzugt ein transgener Mikroorganismus ist, umfassend einen Vektor für die Expression von Isopren-Synthase, funktionsfähig gebunden an einen funktionellen Promoter in dem Mikroorganismus.

26. Verfahren zur Erhöhung des Ertrags einer Pflanze, angebaut unter Bedingungen, die periodischen oxidativen Stress bei der Pflanze verursachen, umfassend das Verabreichen während der frühen Ontogenese der Pflanze einer agrobiologischen Zusammensetzung, umfassend Katalase-produzierende Mikroorganismen, wobei die Zusammensetzung ferner Wachstumsnährstoffe für die Mikroorganismen und Substanzen umfasst, die die Katalase-Produktion in den Mikroorganismen herbeiführen, wobei der Mikroorganismus bevorzugt ein transgener Mikroorganismus ist, umfassend einen Vektor zur Expression der Katalase, funktionsfähig gebunden an einen funktionellen Promoter in dem Mikroorganismus, und wobei der Mikroorganismus symbiotisch zur Pflanze ist.

## Revendications

1. Procédé pour augmenter le rendement d'une plante cultivée dans des conditions qui causent un stress oxydatif périodique dans ladite plante, comprenant l'étape de culture d'un semis, une culture de tissu ou un plantule dans une plante, et comprenant en outre l'étape de manipulation de ladite plante ou l'environnement de ladite plante de sorte que ledit stress oxydatif périodique soit intentionnellement prévenu pendant l'ontogenèse précoce de ladite plante afin de maintenir le composant AOX de la respiration à l'obscurité totale dans ladite plante à des taux essentiellement égaux à celui d'une plante qui n'a jamais été exposée au stress oxydatif.

2. Procédé selon la revendication 1, dans lequel lesdites conditions qui causent un stress oxydatif périodique sont choisies parmi un stress de sécheresse périodique, un stress de température périodique, un stress de rayonnement périodique, un stress de sel périodique et une exposition à O₃ périodique.

3. Procédé selon la revendication 1 ou 2, dans lequel ledit composant AOX de la respiration à l'obscurité totale dans ladite plante est proche de zéro.

4. Procédé selon l'une quelconque des revendications 1 à 3, dans lequel ledit stress oxydatif est intentionnellement prévenu en contrôlant le contenu d'une espèce d'oxygène réactif dans l'environnement de culture.

5. Procédé selon l'une quelconque des revendications 1 à 4, dans lequel le stress oxydatif périodique pendant l'ontogenèse précoce de ladite plante est prévenu par exposition des plantes à un taux de CO₂ qui réduit le taux de respiration.

6. Procédé selon la revendication 4 ou 5, dans lequel ladite espèce d'oxygène réactif dans l'environnement de culture est O₃ dans l'atmosphère de croissance.

7. Procédé selon l'une quelconque des revendications 3 à 6, dans lequel ladite exposition à O₃ est contrôlée en utilisant des épurateurs de O₃ et/ou des épurateurs de NOx.

8. Procédé selon l'une quelconque des revendications 3 à 6, dans lequel ladite exposition à O₃ est contrôlée par exposition de ladite plante ou son atmosphère de croissance à de l'isoprène produit de façon exogène ou endogène, de préférence dans lequel ledit isoprène est produit de façon exogène par des micro-organismes éventuellement transgéniques ou recombinants capable d'émettre de l'isoprène.

9. Procédé selon l'une quelconque des revendications 1 à 8, dans lequel l'ontogenèse précoce de ladite plante est le stade préfloral ou le stade végétatif, de préférence une période de 1 à 6 mois post-germination.

10. Procédé selon l'une quelconque des revendications 1 à 9, dans lequel ladite plante est une plante pérenne ou une plante cultivée, de manière préférée entre toutes le blé, le maïs, le soja, la pomme de terre, le riz, la canne à sucre, la betterave sucrière, l'onagre, la limnanthe, le houblon, le jojoba, l'arachide, le carthame, l'orge, l'avoine, le seigle, le blé, le sorgho, le tabac, le kapok, les fèves, les lentilles, les pois, le soja, le colza, la moutarde, le pavot, les olives, les tournesols, la noix de coco, le ricin, les fèves de cacao, les arachides, le coton, le lin, le chanvre, le jute, la cannelle, le camphre, le café, la canne à sucre, le thé, et une plante de caoutchouc naturel.

11. Procédé selon l'une quelconque des revendications 1 à 10, dans lequel ledit procédé comprend en outre l'étape d'interruption de la manipulation de ladite plante ou l'environnement de ladite plante lorsque la phase ontogénique de ladite plante est terminée.

12. Procédé selon l'une quelconque des revendications 1 à 11, dans lequel ledit procédé comprend en outre la détermination avant ou simultanément à la culture dudit semis, la culture de tissu ou le plantule dans une plante de :
- la respiration à l'obscurité totale et/ou la respiration via la voie d'oxydase alternative (AOX) dans une plante de la même variété ;
- la durée de la phase ontogénique dans une plante de la même variété ; et/ou
- la durée et l'intervalle des conditions périodiques qui causent un stress oxydatif ;
et l'utilisation desdites informations afin de prévenir intentionnellement l'exposition de ladite plante audit stress oxydatif périodique pendant l'ontogenèse précoce de ladite plante, mais pas pendant la phase post-ontogénique, de préférence dans lequel lesdites informations sont utilisées pour sélectionner la date de semis ou de (trans)plantation pour ladite plante afin de prévenir essentiellement l'exposition de ladite plante audit stress oxydatif périodique pendant l'ontogenèse précoce de ladite plante.

13. Procédé selon l'une quelconque des revendications 1 à 12, dans lequel ladite ontogenèse précoce de ladite plante est la phase végétative ou préflorale, et dans lequel la phase post-ontogénique est la phase générative.

14. Procédé pour augmenter la production de culture annuelle moyenne d'une zone de production de culture, comprenant la culture d'une plante selon l'une quelconque des revendications 1 à 13 et en utilisant ladite plante en tant que plante cultivée dans ladite zone de production, de préférence dans lequel ladite augmentation de la production de culture annuelle moyenne est induite par une efficacité de fixation de carbone nette moyenne, production de biomasse, teneur en matières sèches et/ou résistance aux organismes nuisibles améliorées de ladite plante cultivée.

15. Plante pouvant être obtenue par le procédé selon l'une quelconque des revendications 1 à 13, ladite plante présentant une respiration de base ou une respiration via la voie d'oxydase alternative (AOX) qui est significativement inférieure à celle d'une plante de la même variété cultivée dans les mêmes conditions périodiques qui causent un stress oxydatif mais non cultivées par le procédé de l'une quelconque des revendications 1 à 13, ladite plante étant une plante adulte, et ladite plante présentant un premier taux de respiration sous ozone ambiant qui est essentiellement égal au taux de respiration à l'obscurité présenté par ladite plante pendant l'ontogenèse précoce, et ladite plante, après exposition temporaire aux conditions ambiantes plus 100 ppbv d'ozone, présentant un deuxième taux de respiration, ledit deuxième taux étant une augmentation significative par rapport audit premier taux, et, après ladite exposition temporaire, ledit deuxième taux de respiration retournant à des taux pré-exposition, de préférence ladite ontogenèse précoce étant la période entre la germination et de 0,5 à 6 mois post-germination ou ladite ontogenèse foliaire précoce étant la période entre la levée et 15 à 60 jours après l'apparition des premières feuilles.

16. Plante selon la revendication 15, la plante étant un non-émetteur d'isoprène ou un émetteur faible d'isoprène.

17. Plante selon l'une quelconque des revendications 15 à 16, ladite plante étant une plante pérenne ou une plante cultivée, de manière préférée entre toutes le blé, le maïs, le soja, la pomme de terre, le riz, la canne à sucre, la betterave sucrière, l'onagre, la limnanthe, le houblon, le jojoba, l'arachide, le carthame, l'orge, l'avoine, le seigle, le blé, le sorgho, le tabac, le kapok, les fèves, les lentilles, les pois, le soja, le colza, la moutarde, le pavot, les olives, les tournesols, la noix de coco, le ricin, les fèves de cacao, les arachides, le coton, le lin, le chanvre, le jute, la cannelle, le camphre, le café, la canne à sucre, le thé, et une plante de caoutchouc naturel.

18. Plante selon l'une quelconque des revendications 15 à 17, ladite plante présentant un taux de respiration via la voie d'oxydase alternative (AOX) qui est inférieur à 30, de préférence inférieur à 28, 27, 26 ou 25 % de la respiration à l'obscurité totale.

19. Partie de plante obtenue à partir d'une plante selon l'une quelconque des revendications 15 à 18, ladite partie de plante présentant une respiration de base ou une respiration via la voie d'oxydase alternative (AOX) qui est significativement inférieure à une partie de plante d'une plante de la même variété cultivée dans les mêmes conditions périodiques qui causent un stress oxydatif mais non cultivées par le procédé de l'une quelconque des revendications 1 à 13, ladite partie de plante présentant un premier taux de respiration sous ozone ambiant qui est essentiellement égal au taux de respiration à l'obscurité présenté par la plante à partir de laquelle la partie est obtenue pendant l'ontogenèse précoce, et ladite partie de plante après exposition temporaire aux conditions ambiantes plus 100 ppbv d'ozone présente un deuxième taux de respiration, ledit deuxième taux étant une augmentation significative par rapport audit premier taux, et après ladite exposition temporaire, ledit deuxième taux de respiration retourne aux taux pré-exposition, ladite ontogenèse précoce étant de préférence la période entre la germination et 0,5 à 6 mois post-germination ou ladite ontogenèse foliaire précoce étant la période entre la levée et 15 à 60 jours après l'apparition des premières feuilles.

20. Utilisation d'une serre pour cultiver des plantes pour contrôler la teneur d'une espèce d'oxygène réactif pour prévenir le stress oxydatif périodique pendant l'ontogenèse précoce desdites plantes, ladite espèce d'oxygène réactif étant de préférence O₃.

21. Utilisation selon la revendication 20, ladite serre étant équipée d'épurateurs de O₃ afin de maintenir le composant AOX de la respiration à l'obscurité totale dans ladite plante à des taux pratiquement égaux à ceux d'une plante qui n'a jamais été exposée au stress oxydatif.

22. Procédé pour optimiser la production d'un type spécifique de plante sur une zone cultivée sélectionnée, comprenant les étapes de :
a) détermination pour ledit type de plante spécifique de la phase ontogénique précoce dans laquelle le taux de respiration de base ou le taux de respiration via la voie d'oxydase alternative (AOX) est fixé à un niveau minimal permanent ;
b) surveillance dans l'air sur ladite zone cultivée de la concentration d'espèce d'oxygène réactif ; et
c) semis d'une semence ou plantation d'un semis ou plantule dudit type spécifique de plante et ensuite laisser le semis ou plantule se développer en une plante, où, pendant ladite phase ontogénique précoce de ladite plante, une exposition à des concentrations indésirables d'espèce d'oxygène réactif dans l'air sur ladite zone cultivée est essentiellement prévenue par sélection du temps de semis ou de plantation en utilisant les données obtenues dans l'étape b).

23. Utilisation d'une composition agrobiologique dans un procédé pour augmenter le rendement d'une plante cultivée dans des conditions qui causent un stress oxydatif périodique dans ladite plante, comprenant l'administration de ladite composition agrobiologique pendant l'ontogenèse précoce de ladite plante, ladite composition agrobiologique comprenant des micro-organismes émetteurs d'isoprène, ladite composition comprenant en outre des nutriments de croissance pour ledit micro-organisme et des substances qui induisent la production d'isoprène dans ledit micro-organisme, ledit micro-organisme étant de préférence un micro-organisme transgénique comprenant un vecteur pour l'expression d'isoprène synthase, fonctionnellement lié à un promoteur fonctionnel dans ledit micro-organisme.

24. Utilisation d'une composition agrobiologique dans un procédé pour augmenter le rendement d'une plante cultivée dans des conditions qui causent un stress oxydatif périodique dans ladite plante, comprenant l'administration de ladite composition agrobiologique pendant l'ontogenèse précoce de ladite plante, ladite composition agrobiologique comprenant des micro-organismes producteurs de catalase, ladite composition comprenant en outre des nutriments de croissance pour ledit micro-organisme et des substances qui induisent la production de catalase dans ledit micro-organisme, ledit micro-organisme étant de préférence un micro-organisme transgénique comprenant un vecteur pour l'expression de catalase, fonctionnellement lié à un promoteur fonctionnel dans ledit micro-organisme, et ledit micro-organisme étant symbiotique pour ladite plante.

25. Procédé pour augmenter le rendement d'une plante cultivée dans des conditions qui causent un stress oxydatif périodique dans ladite plante, comprenant l'administration pendant l'ontogenèse précoce de ladite plante d'une composition agrobiologique comprenant des micro-organismes émetteurs d'isoprène, ladite composition comprenant facultativement en outre des nutriments de croissance pour ledit micro-organisme et des substances qui induisent la production d'isoprène dans ledit micro-organisme, ledit micro-organisme étant de préférence un micro-organisme transgénique comprenant un vecteur pour l'expression d'isoprène synthase, fonctionnellement lié à un promoteur fonctionnel dans ledit micro-organisme.

26. Procédé pour augmenter le rendement d'une plante cultivé dans des conditions qui causent un stress oxydatif périodique dans ladite plante, comprenant l'administration pendant l'ontogenèse précoce de ladite plante d'une composition agrobiologique comprenant des position producteurs de catalase, ladite composition comprenant facultativement en outre des nutriments de croissance pour ledit micro-organisme et des substances qui induisent la production de catalase dans ledit micro-organisme, ledit micro-organisme étant de préférence un micro-organisme transgénique comprenant un vecteur pour l'expression de catalase, fonctionnellement lié à un promoteur fonctionnel dans ledit micro-organisme, et ledit micro-organisme étant symbiotique pour ladite plante.
